(19) Europäisches Patentamt
European Patent Office
Office européen des brevets

(11) Veröffentlichungsnummer: **0 563 732 A1**

# (12) EUROPÄISCHE PATENTANMELDUNG

(21) Anmeldenummer: **93104660.1**

(22) Anmeldetag: **22.03.93**

(51) Int. Cl.[5]: **C07D 498/06**, A61K 31/535, //(C07D498/06,265:00,221:00)

(30) Priorität: **02.04.92 DE 4210942**

(43) Veröffentlichungstag der Anmeldung: **06.10.93 Patentblatt 93/40**

(84) Benannte Vertragsstaaten: **AT BE CH DE DK ES FR GB GR IE IT LI LU MC NL PT SE**

(71) Anmelder: **BAYER AG**
**D-51368 Leverkusen(DE)**

(72) Erfinder: **Schneider, Stephan, Dr.**
**116 Acorn Road**
**Madison, CT 06443(US)**
Erfinder: **Bartel, Stephan, Dr.**
**Margaretenhöhe 7**
**W-5060 Bergisch Gladbach 2(DE)**
Erfinder: **Ruppelt, Martin, Dr.**
**Lützenberger Strasse 351**
**W-5600 Wuppertal 1(DE)**
Erfinder: **Schriewer, Michael, Dr.**
**Am Thelen Siefen 1a**
**W-5068 Odenthal(DE)**
Erfinder: **Schulze, Thomas J., Dr.**
**Jakob-Böhme-Strasse 11**
**W-5000 Köln 80(DE)**
Erfinder: **Neumann, Rainer, Dr.**
**Albert-Einstein-Strasse 27**
**W-5024 Pulheim 2(DE)**

(54) **7-Oxo-7H-pyrido 1,2,3-de 1,4 benzoxazin-6-carbonsäuren und -ester und ihre Verwendung als antivirale Mittel.**

(57) Die vorliegende Erfindung betrifft 7-Oxo-7H-pyrido[1,2,3-d,e][1,4]benzoxacin-6-carbonsäuren und -ester der allgemeinen Formel (I), Verfahren zu ihrer Herstellung und ihre Verwendung als Arzneimittel, insbesondere als antiviral wirksame Arzneimittel.

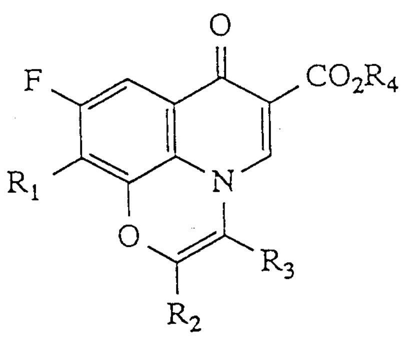

(I)

EP 0 563 732 A1

Die vorliegende Erfindung betrifft 7-Oxo-7H-pyrido[1,2,3-de][1,4]benzoxacin-6-carbonsäuren und -ester, Verfahren zu ihrer Herstellung und ihre Verwendung als Arzneimittel, insbesondere als antiviral wirksame Arzneimittel.

Aus der EP-A-253 235 sind 1,8-verbrückte 4-Chinoloncarbonsäuren mit einer antibakteriellen Wirkung bekannt geworden.

Die vorliegende Erfindung betrifft 7-Oxo-7H-pyrido[1,2,3-de][1,4]benzoxacin-6-carbonsäuren und -ester der allgemeinen Formel (I)

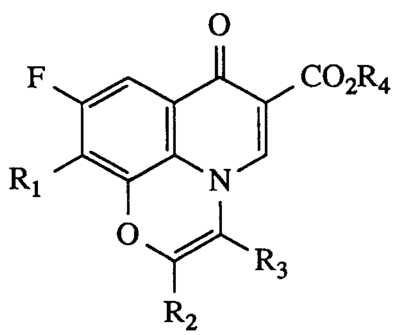

(I),

in welcher

R¹ für 1-Piperidinyl oder für einen Rest der Formel

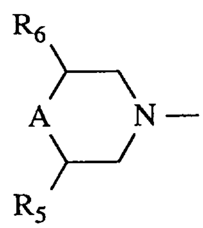

oder

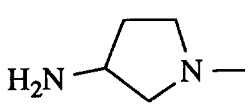

steht,
worin

A eine Gruppe der Formel $-NR^7$, ein Sauerstoffatom oder die $-CH_2$-Gruppe bedeutet,
worin

$R^7$ Wasserstoff oder geradkettiges oder verzweigtes Alkyl mit bis zu 6 Kohlenstoffatomen bedeutet, das gegebenenfalls durch Hydroxy oder Phenyl substituiert ist,

$R^5$ und $R^6$ gleich oder verschieden sind und Wasserstoff, Cyclohexyl, Phenyl oder geradkettiges oder verzweigtes Alkyl mit bis zu 4 Kohlenstoffatomen bedeuten,

$R^2$ für Wasserstoff, oder
für geradkettiges oder verzweigtes Pefluoralkyl mit bis zu 4 Kohlenstoffatomen steht, oder
für geradkettiges oder verzweigtes Alkyl mit bis zu 6 Kohlenstoffatomen steht, das gegebenenfalls durch Phenyl, Hydroxy, Halogen, geradkettiges oder verzweigtes Alkoxy oder Alkylthio mit jeweils bis zu 3 Kohlenstoffatomen substituiert ist,
für Phenyl, Pyridyl, Furyl oder Thienyl steht, die gegebenenfalls einfach durch Halogen

oder durch geradkettiges oder verzweigtes Alkyl oder Alkoxy mit jeweils bis zu 3 Kohlenstoffatomen substituiert sind,

$R^3$ für Wasserstoff, Phenyl, Trifluormethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen steht,

und deren Hydrate und Salze, gegebenenfalls in einer isomeren Form, mit Ausnahme von 9-Fluor-3-methy-10-(1-piperazinyl)-7-oxo-7H-pyrido[1,2,3-de][1,4]-benzoxacin-6-carbonsäure und deren 3- und 4-Methyl-1-piperazinyl-Derivaten, 9-Fluor-10-(4-methyl-1-piperazinyl)-7-oxo-7H-pyrido[1,2,3-d,e][1,4]benzoxacin-6-carbonsäure und deren 2-Methyl-Derivat.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Alkali-, Erdalkali, Silber-und Guanidiniumsalze der erfindungsgemäßen Verbindungen sein.

Bevorzugt sind erfindungsgemäße Verbindungen der allgemeinen Formel (I),
in welcher

$R^1$ für 1-Piperidinyl oder für einen Rest der Formel

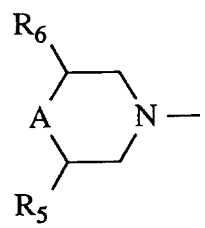

oder

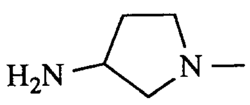

steht,
worin

A eine Gruppe der Formel $-NR^7$, ein Sauerstoffatom oder die $-CH_2$-Gruppe bedeutet, worin

$R^7$ Wasserstoff oder geradkettiges oder verzweigtes Alkyl mit bis zu 4 Kohlenstoffatomen bedeutet, das gegebenenfalls durch Hydroxy oder Phenyl substituiert ist,

$R^5$ und $R^6$ gleich oder verschieden sind und Wasserstoff, Cyclohexyl, Phenyl oder geradkettiges oder verzweigtes Alkyl mit bis zu 4 Kohlenstoffatomen bedeuten,

$R^2$ für Wasserstoff oder
für geradkettiges oder verzweigtes Perfluoralkyl mit bis zu 4 Kohlenstoffatomen steht,
für geradkettiges oder verzweigtes Alkyl mit bis zu 5 Kohlenstoffatomen steht, das gegebenenfalls durch Phenyl, Hydroxy, Fluor, Chlor oder Alkoxy oder Alkylthio mit jeweils bis zu 2 Kohlenstoffatomen substituiert ist,
für Phenyl, Pyridyl, Furyl oder Thienyl steht, die gegebenenfalls einfach durch Fluor, Chlor, Brom oder geradkettiges oder verzweigtes Alkyl oder Alkoxy mit jeweils bis zu 3 Kohlenstoffatomen substituiert sind,

$R^3$ für Wasserstoff, Phenyl, Trifluormethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen

men steht

und deren Hydrate und Salze, gegebenenfalls in einer isomeren Form.

Besonders bevorzugt sind erfindungsgemäße Verbindungen der allgemeinen Formel (I), in welcher

$R^1$ für 1-Piperidinyl steht, oder für einen Rest der Formel

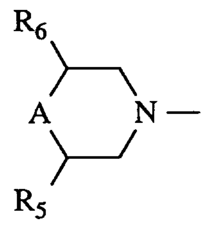

oder

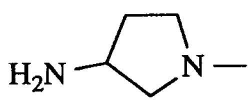

steht, worin

A eine Gruppe der Formel $-NR^7$, ein Sauerstoffatom oder die $-CH_2$-Gruppe bedeutet, worin

$R^7$ Wasserstoff oder geradkettiges oder verzweigtes Alkyl mit bis zu 4 Kohlenstoffatomen bedeutet, das gegebenenfalls durch Hydroxy oder Phenyl substituiert ist,

$R^5$ und $R^6$ gleich oder verschieden sind und Wasserstoff, Cyclohexyl, Phenyl oder geradkettiges oder verzweigtes Alkyl mit bis zu 4 Kohlenstoffatomen bedeuten,

$R^2$ für Wasserstoff oder für geradkettiges oder verzweigtes Perfluoralkyl mit bis zu 4 Kohlenstoffatomen steht, für geradkettiges oder verzweigtes Alkyl mit bis zu 5 Kohlenstoffatomen steht, das gegebenenfalls durch Phenyl, Hydroxy, Fluor, Chlor, Methoxy, Ethoxy oder Methylthio substituiert ist, für Phenyl, Pyridyl oder Furyl steht, die gegebenenfalls einfach durch Fluor, Chlor, Brom, Methyl, Ethyl, Methoxy oder Ethoxy substituiert sind,

$R^3$ für Wasserstoff, Phenyl, Trifluormethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen steht

und deren Hydrate und Salze, gegebenenfalls in einer isomeren Form.

Außerdem wurde ein Verfahren zur Herstellung der erfindungsgemäßen Verbindungen der allgemeinen Formel (I) gefunden, dadurch gekennzeichnet, daß man

[A] Verbindungen der allgemeinen Formel (II)

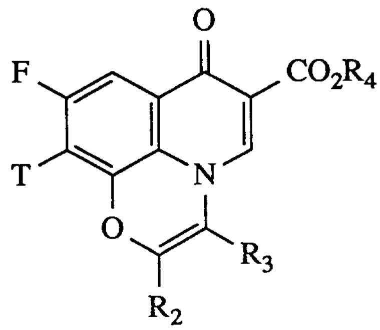

(II)

in welcher

$R^2$ und $R^3$ die oben angegebene Bedeutung haben,

$R^4$ die oben angegebene Bedeutung hat, aber vorzugsweise für Wasserstoff steht,

und

T für Halogen, vorzugsweise für Fluor steht,

mit Verbindungen der allgemeinen Formel (III)

$R^1$-H (III),

in welcher

$R^1$ die oben angegebene Bedeutung hat,

in inerten Lösemitteln, in Anwesenheit einer Base unter Schutzgasatmosphäre umsetzt, oder

[B] im Fall, daß $R^2$ und $R^3$ für Wasserstoff stehen,

auch Aldehyde der allgemeinen Formel (IV)

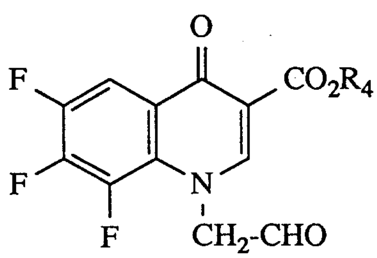

(IV),

in welcher

$R^4$ die oben angegebene Bedeutung hat aber vorzugsweise für Wasserstoff steht,

entweder zunächst mit Verbindungen der allgemeinen Formel (III) oder (IIIa), vorzugsweise mit (IIIa)

$R^1$-H (III),

W-H (IIIa),

in welcher

$R^1$ die oben angegebene Bedeutung hat,

und

W die oben angegebene Bedeutung von $R^1$ hat, wobei aber eine der cyclischen Aminfunktionen durch eine Schutzgruppe, vorzugsweise durch Methoxycarbonyl, Ethoxycarbonyl oder tert. Butoxycarbonyl, geschützt ist,

in inerten Lösemitteln, in Anwesenheit einer Base, unter gleichzeitiger Cyclisierung umsetzt, die Schutzgruppe nach üblicher Methode abspaltet und anschließend je nach gewünschter Bedeutung von $R^4$ entweder eine Veresterung oder Verseifung nach üblicher Methode durchführt.

Das erfindungsgemäße Verfahren kann durch folgendes Formelschema beispielhaft erläutert werden:

[A]

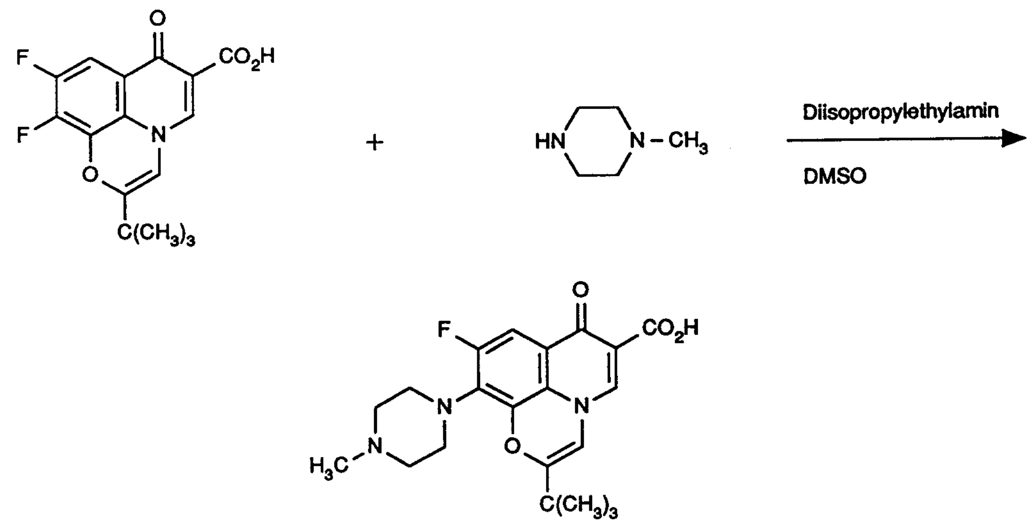


[B]

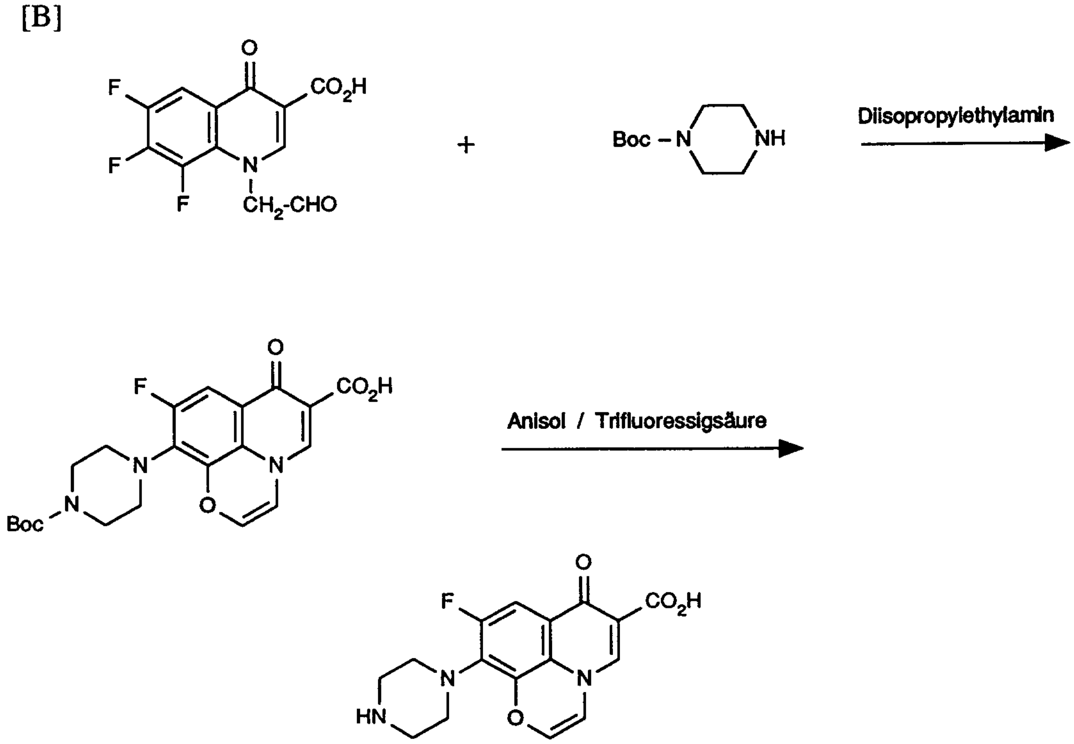


Schutzgruppe im Rahmen der Erfindung sind die aus der Peptid-Chemie bekannten Gruppen wie beispielsweise Benzyloxycarbonyl, Methoxycarbonyl, Ethoxycarbonyl, Propoxycarbonyl, Isopropoxycarbonyl, Butoxycarbonyl oder Isobutoxycarbonyl. Bevorzugt sind Methoxy- und Ethoxycarbonyl.

Als Lösemittel eignen sich für alle Verfabrensschritte die üblichen inerten Lösemittel, die sich unter den Reaktionsbedingungen nicht verändern. Hierzu gehören bevorzugt organische Lösemittel wie Ether z.B. Diethylether, Glykolmono-oder -dimethylether, Dioxan oder Tetrahydrofuran, oder Kohlenwasserstoffe wie Benzol, Toluol, Xylol, Cyclohexan oder Erdölfraktionen, oder Halogenkohlenwasserstoffe wie Methylenchlorid, Chloroform, Tetrachlorkohlenstoff, oder Dimethylsulfoxid, N,N-Dimethylformamid, Hexamethylphosphorsäuretriamid, Sulfolan, Essigester, Pyridin, Triethylamin, N-Methylpyrrolidin, Anisol oder Picolin. Ebenso ist

es möglich, Gemische der genannten Lösemittel zu verwenden. Bevorzugt sind Dimethylsulfoxid und N,N-Dimethylformamid.

Als Basen eignen sich die üblichen basischen Verbindungen. Hierzu gehören beispielsweise Alkali- oder Erdalkalihydroxide, Pyridin, Triethylamin, Diisopropylethylamin oder N-Methylpiperidin, oder bicyclische Amidine wie 1,5-Diazabicyclo[3,4,0]-nonene-5 (DBN), 1,5-Diazabicyclo[3,4,0]undecene-5 (DBU) oder DABCO. Bevorzugt sind Triethylamin, Diisopropylethylamin und DABCO.

Die Basen werden im allgemeinen in einer Menge von 1 bis 3 mol, bevorzugt von 1 bis 1,5 mol, bezogen auf 1 mol der entsprechenden Carbonsäure eingesetzt.

Die Verfahren [A] und [B] werden im allgemeinen in einem Temperaturbereich von +0 °C bis +150 °C, bevorzugt von +0 °C bis +120 °C durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel eignen sich für die Verseifung Wasser oder Wasser in Kombination mit einem üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid, oder Essigsäure. Besonders bevorzugt werden Alkohole wie Methanol, Ethanol, Propanol oder Isopropanol verwendet.

Die Verseifung erfolgt mit Säuren wie beispielsweise Chlorwasserstoffsäure, Essigsäure, Bromwasserstoffsäure, Methansulfonsäure, Schwefelsäure oder Perchlorsäure.

Die Verseifung wird im allgemeinen in einem Temperaturbereich von 0 °C bis 130 °C, bevorzugt von 20 °C bis 110 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Säure im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verseifung von tert.Butylestern erfolgt im allgemeinen mit Säuren, wie beispielsweise Salzsäure oder Trifluoressigsäure, in Anwesenheit eines der oben angegebenen Lösemittel und/oder Wasser oder deren Gemische, vorzugsweise mit Dioxan oder Tetrahydrofuran.

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dann der entsprechende Alkohol auch als Lösemittel eingesetzt.

Als Katalysatoren können anorganische Säuren, wie bespielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator bezogen auf 1 mol Reaktionspartner ein.

Die Abspaltung der Aminoschutzgruppen erfolgt nach literaturbekannten Methoden [vgl. Houben-Weyl, "Methoden der organischen Chemie", Synthese von Peptiden II, 4. Auflage, Bd. 15/1,15/2, Georg Thieme Verlag], bevorzugt mit Trifluoressigsäure in Anisol.

Die Verbindungen der allgemeinen Formel (II) sind teilweise bekannt [vgl. hierzu EP 253 235 A1; J. Heterocycl. Chem. 28, 1067 (1991)] oder neu und können beispielsweise hergestellt werden, indem man Verbindungen der allgemeinen Formel (V)

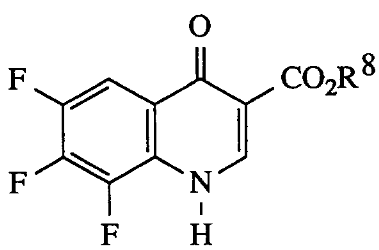

(V),

in welcher

$R^8$ für einen $C_1$-$C_4$-Alkylrest steht,

mit einer $\alpha$-Halogencarbonylverbindung der allgemeinen Formel (VI)

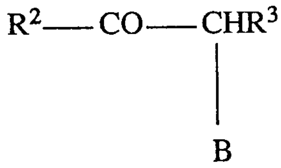

(VI),

in welcher

$R^2$ und $R^3$ die oben angegebene Bedeutung haben

und

B für Halogen, vorzugsweise für Brom steht,

in einem der oben aufgeführten Lösemitteln und Basen, vorzugsweise Dimethylformamid und Kaliumcarbonat umsetzt,

oder

indem man Verbindungen der allgemeinen Formel (VII)

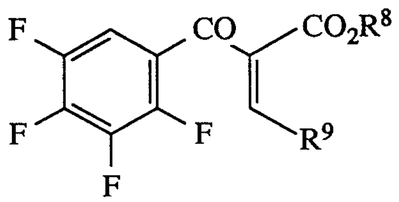

(VII),

in welcher

$R^8$ die oben angegebene Bedeutung hat

und

$R^9$ für $C_1$-$C_4$-Alkoxy steht,

mit Verbindungen der allgemeinen Formel (VIII)

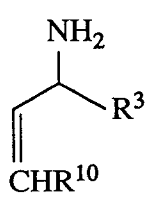

(VIII),

in welcher

$R^3$ die oben angegebene Bedeutung hat

und

$R^{10}$ für Wasserstoff oder $C_1$-$C_4$-Alkyl steht,

in einem der oben aufgeführten Lösemitteln, vorzugsweise Ethanol, und gegebenenfalls in Anwesenheit einer dort ebenfalls aufgeführten Aminbase, umsetzt und anschließend mit Dimethoxyethan und Natriumhydrid in die Verbindungen der allgemeinen Formel (IX)

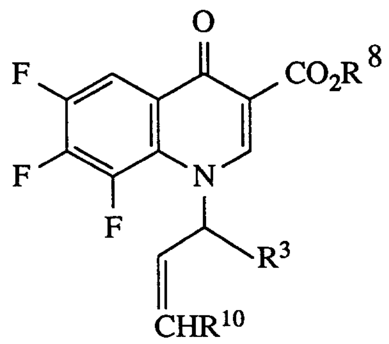

(IX),

in welcher

$R^8$, $R^3$ und $R^{10}$ die oben angegebenen Bedeutungen haben,

überführt,

anschließend durch Ozonolyse nach üblichen Methoden die Doppelbindung zur Carbonylfunktion spaltet, und in einem letzten Schritt die Cyclisierung wie oben beschrieben durchführt, und im Fall der unter $R^2$ und $R^3$ oben angegebenen weiteren Substituenten, diese nach üblichen Methoden variiert.

Die Umsetzung mit den Verbindungen der allgemeinen Formel (VIII) erfolgt in einem Temperaturbereich von -20 °C bis +30 °C, vorzugsweise von 0 °C bis Raumtemperatur und Normaldruck.

Die Ozonolyse wird im allgemeinen bei -78 °C in einem Lösemittelgemisch Methanol/Methylenchlorid und unter Schutzgasatmosphäre durchgeführt.

Die Verbindungen der allgemeinen Formeln (VII) und (VIII) sind teilweise bekannt oder können nach üblichen Methoden hergestellt werden.

Die Verbindungen der allgemeinen Formel (IX) sind größtenteils neu und können nach dem oben beschriebenen Verfahren hergestellt werden.

Die Verbindungen der allgemeinen Formeln (V) und (VI) sind an sich bekannt [vgl. EP-A-253 235] oder können nach bekannten Methoden hergestellt werden.

Ebenso sind die Verbindungen der allgemeinen Formeln (III) und (IIIa) bekannt, teilweise käuflich oder nach üblichen Methoden herstellbar.

Die Aldehyde der allgemeinen Formel (IV) sind an sich bekannt oder können nach publizierten Methoden hergestellt werden [vgl. EP-A- 253 235].

Die erfindungsgemäßen Verbindungen zeigen ein weiteres nicht vorhersehbares, wertvolles pharmakologisches Wirkspektrum. Sie sind überraschenderweise geeignet zur Behandlung von virusinduzierten Erkrarkungen.

Ebenso können die Zwischenprodukte der allgemeine Formel (II) dieses Wirkspektrum aufweisen.

Der Nachweis wurde in mit Hepatitis B Virus DNA transfizierten Hepatomzellen (HEP G2.2.15) geführt. Die Ergebnisse der unten aufgeführten Beispiele wurden zu dem in der folgenden Literaturangabe [Sells, M.A.; Chen, M.L., Acs, G., Proc. Natl. Acad. Sci. USA S. 1005 - 1009, Vol. 84 (1987] beschriebenen HBV-Testsystem ermittelt:

Transfizierte Hepatomzellen (HEP G2.2.15) wurden mit verschiedenen Konzentrationen an der jeweiligen Verbindung inkubiert. Durch Behandlung der transfizierten Hepatomzellinie HEP G2.2.15 mit den erfindungsgemäßen Verbindungen konnte das Auftreten der virusspezifischen HBV-DNA im Überstand sowie eine Reduktion des $HB_sAg$ Spiegels gezeigt werden. Es wurde weiter gefunden, daß die erfindungsgemäßen Verbindungen zu einer Reduktion der replikativen Intermediate der Hepatitisviren führen. Im Überstand der Zellkulturen wurde nach PEG Fällung der Gehalt an HBV-DNA bestimmt. Dies geschah unter Verwendung einer nicht radioaktiv markierten HBV genomischen DNA Probe [Pauly, P., 1982, Ph.D. Thesis, University of Göttingen, F.R.G.; Köchel et al., 1990, EMBL, Accession-Nr. X 51790]. Gleichzeitig erfolgte der Nachweis der Beeinflussung der $HB_sAg$ Bildung mittels eines kommerziell erhältlichen Elisa Tests. Die angegebenen $IC_{50}$-Werte beziehen sich auf die Substratkonzentrationen, die unter den oben aufgeführten Testbedingungen eine 50%ige Hemmung der HBV-DNA Konzentration im Überstand bewirken.

Tabelle A

| Bsp. Nr. | $IC_{50}$ (µM) | SI |
|---|---|---|
| 1 | 0,1 | >100 |
| 33 | 0,5 | >20 |
| 38 | 5 | ~5 |
| 39 | 0,1 | >100 |
| 16 | 5 | ~20 |

Gleichzeitig erfolgte die Bestimmung des möglicherweise vorhandenen cytotoxischen Einflusses der erfindungsgemäßen Verbindungen mittels Kristallviolettfärbung bzw. über den Einbau von radioaktiv markiertem Thymidin in die zelluläre DNA.

Die Wirkung der erfindungsgemäßen Verbindungen auf andere Zellen wurde durch Inkubation von HEL-, MEF-Zellen getestet. Es zeigte sich keine Beeinflussung der Vitalität dieser Zellen bis zu einer Konzentration von 250 µM.

Als Indikationsgebiete für die erfindungsgemäß verwendbaren Verbindungen können beispielsweise genannt werden:

Die Behandlung von akuten und chronischen Virusinfektionen, die zu einer Hepatitis führen können, beispielsweise die Infektionen mit Hepatitisviren; ferner Virusinfektionen mit Herpesviren Typ 1, 2, Zytomegaloviren, Varizella-Zoster-Viren und HIV.

Besonders bevorzugt ist die Behandlung von chronischen Hepatitis B Virusinfektionen und die Behandlung von akuter Hepatitis-B Virusinfektion.

Zur vorliegenden Erfindung gehören pharmazeutische Zubereitungen, die neben nicht-toxischen, inerten pharmazeutisch geeigneten Trägerstoffen eine oder mehrere Verbindungen der Formeln (I) und/oder (II) enthalten oder die aus einem oder mehreren Wirkstoffen der Formeln (I) und/oder (II) bestehen, sowie Verfahren zur Herstellung dieser Zubereitungen.

Die Verbindungen der Formeln (I) und (II) sollen in den oben aufgeführten pharmazeutischen Zubereitungen, in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formeln (I) und (II) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

9,10-Difluor-2-(2-methoxy-phenyl)-7-oxo-7H-pyrido[1,2,3-de][1,4]benzoxacin-6-carbonsäureethylester

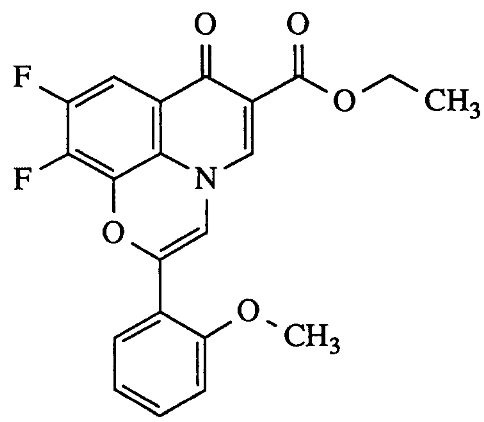

Unter Argon erhitzt man 0,7 g (2,58 mmol) 6,7,8-Trifluor-4-hydroxy-3-chinolin-carbonsäure-ethylester, 0,892 g (6,45 mmol) $K_2CO_3$ und 1,183 g (5,16 mmol) 2-Brom-2'-methoxy-acetophenon in 40 ml abs. DMF 6 h auf 90 °C. Das Lösemittel wird im Vakuum entfernt. Man nimmt mit $CH_2Cl_2$ auf und schüttelt 3 mal mit 50 ml 1N HCl. Die vereinigte organische Phase wird 2 mal mit Wasser gewaschen, über Natriumsulfat getrocknet und im Vakuum eingeengt. Das Rohprodukt wird an Kieselgel (Laufmittel $CH_2Cl_2$ : MeOH = 100:1) gereinigt und aus Toluol umkristallisiert.
Ausbeute: 300 mg (29% der Theorie)
Schmp.: >230 °C

Beispiel II

9,10-Difluor-7-oxo-2-(2-propyl)-7H-pyrido[1,2,3-de][1,4]benzoxacin-6-carbonsäureethylester

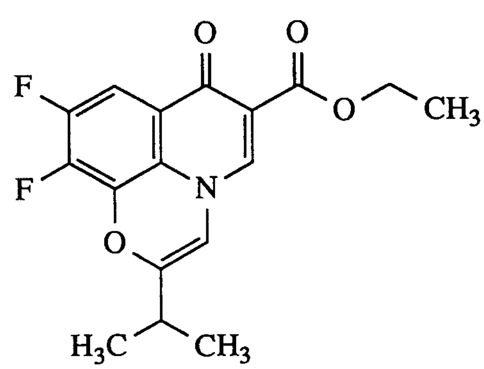

Unter Argon werden 2 g (7,38 mmol) 6,7,8-Trifluor-4-hydroxy-3-chinolin-carbonsäureethylester in 50 ml abs. DMF suspendiert. Man gibt 2,55 g (18,44 mmol) $K_2CO_3$ und 2,43 g (14,75 mmol) 1-Brom-3-methyl-2-butanon in 10 ml abs. DMF zu und erhitzt 8 h auf 90 °C. Man kühlt ab und gießt auf Eiswasser. Das ausgefallene Produkt wird abgesaugt und mit Wasser gewaschen. Man wäscht mit Petrolether und trocknet im Vakuum. Das Rohprodukt wird säulenchromatographisch an Kieselgel (Laufmittel $CH_2Cl_2$: MeOH = 100: 1,5) gereinigt und man erhält 1,5 g (61% der Theorie) der Titelverbindung.
Schmp.: 170 °C

Beispiel III

2-tert.-Butyl-9,10-difluor-7-oxo-7H-pyrido[1,2,3-de][1,4]benzoxacin-6-carbonsäureethylester

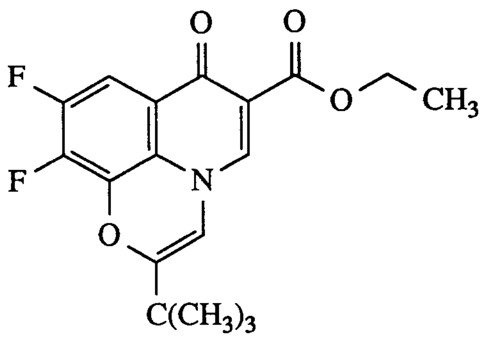

Unter Argon gibt man zu 2 g (7,38 mmol) 6,7,8-Trifluor-4-hydroxy-3-chinolin-carbonsäureethylester in 100 mi abs. DMF 2,55 g (18,44 mmol) $K_2CO_3$ und 2,64 g (14,75 mmol) 1-Brom-3,3-dimethyl-2-butanon und erhitzt 4 h auf 90 °C. Das Lösemittel wird im Vakuum entfernt, der Feststoff mit Wasser verrührt und abfiltriert. Die wäßrige Phase wird mit $CH_2Cl_2$ extrahiert, über $Na_2SO_4$ getrocknet und das Lösemittel im Vakuum entfernt. Das vereinigte Rohprodukt wird an Kieselgel (Laufmittel $CH_2Cl_2$: MeOH = 100 : 1,5) gereinigt. Anschließend wird aus $CHCl_3$ unrkristallisiert.
Ausbeute: 1,24g (48% der Theorie)
Schmp.: 185 °C

Beispiel IV

2-(4-Chlorphenyl)-9,10-difluor-7-oxo-7H-pyrido[1,2,3-de][1,4]benzoxacin-6-carbonsäureethylester

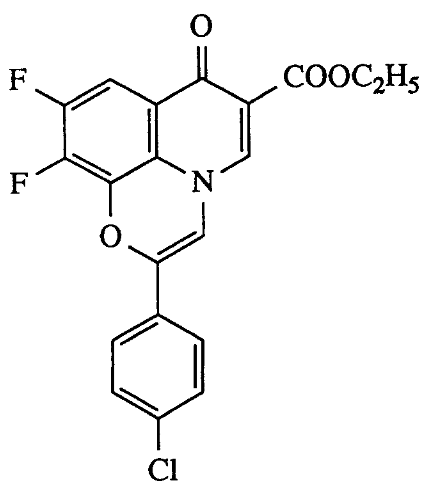

Analog zu Beispiel I wird die Titelverbindung erhalten.
Schmp.: 213 °C

Beispiel V

9,10-Difluor-2-(4-methoxy-phenyl)-7-oxo-7H-pyrido[1,2,3-de][1,4]benzoxacin-6-carbonsäureethylester

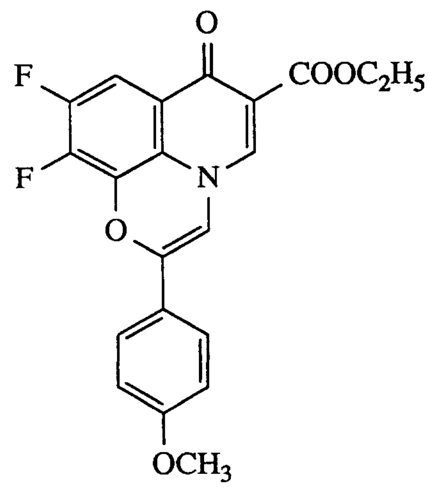

1,9 g (7 mmol) 6,7,8-Trifluor-4-hydroxy-3-chinolin-carbonsäureethylester, 3,2 g (14 mmol) 2-Brom-4'-methoxyacetophenon und 2,43 g (18 mmol) $K_2CO_3$ werden in 14 ml DMF 6 Stunden auf 90 °C erwärmt. Das Lösemittel wird im Vakuum abdestilliert und der Rückstand mit Wasser verrührt. Die Reinigung der Rohprodukts erfolgt durch Säulenchromatographie.
Ausbeute: 2,48 g (89 % d.Th.)
Schmp.: 214 °C

Beispiel VI

2-(tert.-Butyl)-9,10-difluor-7-oxo-7H-pyrido[1,2,3-de][1,4]benzoxacin-6-carbonsäure

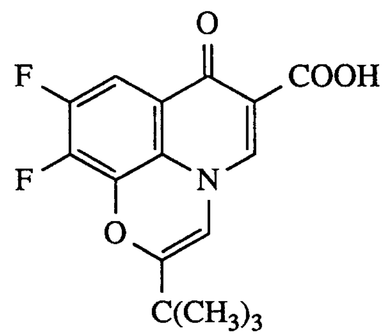

Zu 393 mg (1,12 mmol) der Verbindung aus Beispiel III gibt man 2 ml Essigsäure, 1,3 ml Wasser und 0,17 mi $H_2SO_4$ und kocht 2 h unter Rückfluß. Nach Abkühlen wird mit Wasser versetzt und der Feststoff abfiltriert. Man wäscht mit Wasser nach. Das Produkt wird getrocknet und aus Chloroform unkristallisiert.
Ausbeute: 230 mg (64% der Theorie)
Schmp.: >230 °C

Beispiel VII

9,10-Difluor-2-(2-methoxy-phenyl)-7-oxo-7H-pyrido[1,2,3-de][1,4]benzoxacin-6-carbonsäure

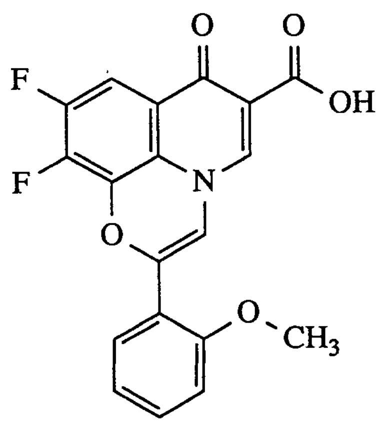

Zu 500 mg (1,25 mmol) der Verbindung aus Beispiel I gibt man 9 ml Essigsäure, 6 ml Wasser und 0,8 ml $H_2SO_4$ und kocht 3 h unter Rückfluß. Nach Abkühlen wird der Feststoff abfiltriert und mit Wasser gewaschen. Das Produkt wird im Hochvakuum getrocknet.

Ausbeute: 450 mg (97% der Theorie)

Schmp.: >230 °C

In Analogie zu den Vorschriften der Beispiele VI und VII werden die in Tabelle I aufgeführten Verbindungen hergestellt:

Tabelle I:

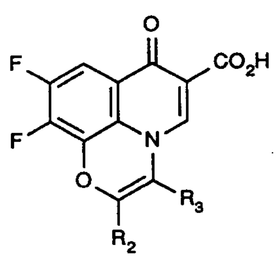

| Bsp.-Nr. | $R^2$ | $R^3$ | F°C |
|---|---|---|---|
| VIII | $-C_6H_4$-p-Cl | H | >300 |
| IX | $-C_6H_4$-p-$OCH_3$ | H | >300 |
| X | $-CH(CH_3)_2$ | H | >230 |

Beispiel XI

10-(4-Butyloxycarbonyl-1-piperazinyl)-9-fluor-7-oxo-7H-pyrido[1,2,3-de][1,4]benzoxacin-6-carbonsäure

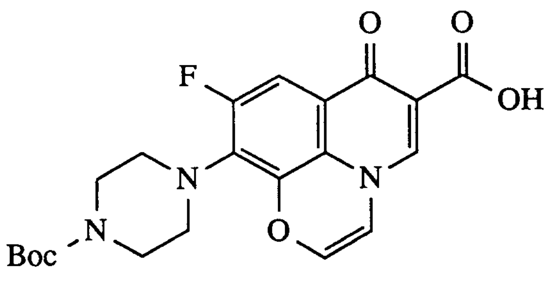

500 mg (1,75 mmol) 1-Formylmethyl-4-oxo-6,7,8-trifluor-4H-chinolin-3-carbonsäure und 652 mg (3,50 mmol) 1-Butyloxycarbonyl-piperazin, werden in einem Gemisch aus 3,0 ml Diisopropylethylamin und 2,5 ml DMSO 4 h unter Rückfluß erhitzt. Nach dem Abkühlen versetzt man mit Ether, dekantiert das Lösemittel ab und verreibt den Rückstand mit Essigester und Ethanol.
Ausbeute: 282 mg (37% der Theorie)
$^1$H-NMR (DMSO-$d_6$): $\delta$ = 6,93 und 7,10 (2d, J = 5 Hz; je 1H, 2-H und 3-H); 7,45 (d, J = 12,5 Hz; 1H, 8-H); 8,75 (s; 1H, 5-H).

Beispiel XII

4H-1-(4-Methyl-1-penten-3-yl)-4-oxo-6,7,8-trifluor-chinolin-3-carbonsäure-ethylester

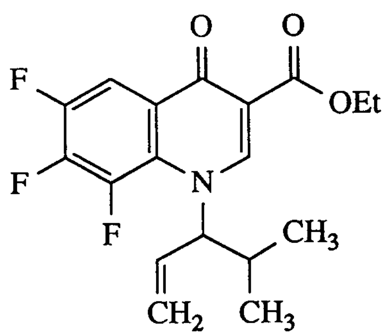

Zu einer Lösung von 4,55 g (14,2 mmol) 3-Ethoxy-2-(2,3,4,5-tetrafluor-benzoyl)-acrylsäure-ethylester und 4,95 ml (28,4 mmol) Diisopropylethylamin in 20 ml Ethanol tropft man bei 0 °C 1,41 g (14,2 mmol) 3-Amino-4-methyl-1-penten in 20 ml Ethanol. Nach 1,5 h bei Raumtemperatur engt man im Vakuum ein, verteilt zwischen Wasser und Methylenchlorid, trocknet die organische Phase mit Natriumsulfat und engt wieder ein.

Der Rückstand wird in 20 ml wasserfreiem Dimethoxyethan gelöst und bei 0 °C zu einer Suspension von 552 mg (18,3 mmol) Natriumhydrid (80 %ig) in 45 ml wasserfreiem Dimethoxyethan getropft. Nach 1 h bei 0 °C fügt man 4 ml Eisessig zu, engt ein und verteilt zwischen Methylenchiorid und Wasser. Die organische Phase wird mit Natriumsulfat getrocknet, eingeengt und an Kieselgel mit Toluol/Essigester chromatographiert.
Ausbeute: 3,35 g (67 %);
Schmp.: 65-67 °C.

Beispiel XIII

4H-4-Oxo-1-(3-phenyl- 1-propen-3-yl)-6,7,8-trifluor-chinolin-3-carbonsäure-ethylester

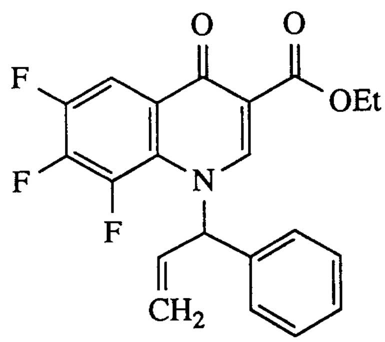

In Analogie zur Vorschrift des Beispiels XII wurde die Titelverbindung aus 3-Amino-3-phenyl-1-propen hergestellt.
Ausbeute: 36 %;
Schmp.: 120 ° C.

Beispiel XIV

4H-4-Oxo-1-(1-penten-3-yl)-6,7,8-trifluor-chinolin-3-carbonsäure-ethylester

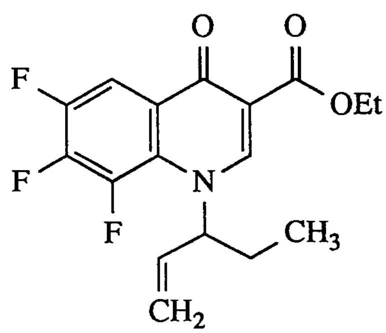

In Analogie zur Vorschrift des Beispiels XII wurde die Titelverbindung aus 3-Amino-1-penten hergestellt.
Ausbeute: 50 %;
Schmp.: 99 ° C.

Beispiel XV

9,10-Difluor-7H-7-oxo-3-(2-propyl)-pyrido[1,2,3-de][1,4]benzoxacin-6-carbonsäure-ethylester

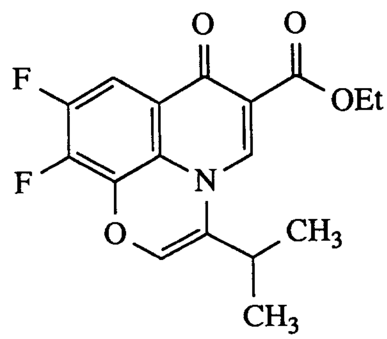

In eine Lösung von 3,35 g (9,5 mmol) der Verbindung aus Beispiel XII in 85 ml Methanol und 21 ml Methylenchlorid leitet man bei -78 °C Ozon bis zur Blaufärbung. Überschüssiges Ozon wird mit Stickstoff vertrieben. Man fügt 7,5 ml Dimethylsulfid zu, läßt auf Raumtemperatur kommen und engt nach ca. 5 h im Vakuum ein. Der Rückstand wird in 50 ml wasserfreiem DMF gelöst, mit 1,31 g (9,5 mmol) $K_2CO_3$ versetzt und 1 h bei Raumtemperatur gerührt. Man fügt 5 ml Eisessig und 500 ml Wasser zu, saugt den ausgefallenen Niederschlag ab, wäscht mit Wasser und trocknet im Vakuum.
Ausbeute: 2,50g (79 %),
NMR: (DMSO-$d_6$): 1,19 (d, J = 7 Hz; 6H, 3'-$CH_3$), 1,28 (t, J = 7 Hz; 3H, 6'-$CH_3$), 2,99 (h, J = 7 Hz; 1H, 3'-H), 4,24 (q, J = 7 Hz; 2H, 6'-$CH_2$), 6,77 (s; 1H, 2-H), 7,56 (dd, J = 8Hz, J = 11 Hz; 1H, 8-H), 8,36 (s; 1H, 5-H).

Beispiel XVI

9,10-Difluor-3-ethyl-7H-7-oxo-pyrido[1,2,3-de][1,4]benzoxacin-6-carbonsäureethylester

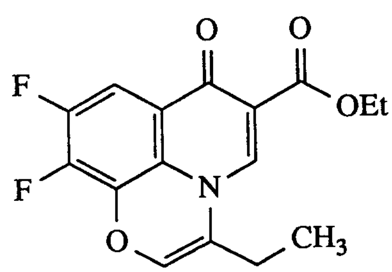

In Analogie zur Vorschrift des Beispiels XV wurde aus der Verbindung des Beispiels XIV die Titelverbindung hergestellt.
Ausbeute: 69 %;
$^1H$-NMR (DMSO-$d_6$): 1,14 und 1,28 (2t, J = 7 Hz; je 3H, 3'-$CH_3$ und 6'-$CH_3$), 4,24 (q,J = 7 Hz; 2H, 6'-$CH_2$), 6,79 (s; 1H, 2-H), 7,55 (dd, J = 8 Hz, J = 11 Hz; 1H, 8-H), 8,28 (s; 1H, 5-H).

Beispiel XVII

9,10-Difluor-7H-7-oxo-3-(2-propyl)-pyrido[1,2,3-de][1,4]benzoxacin-6-carbonsäure

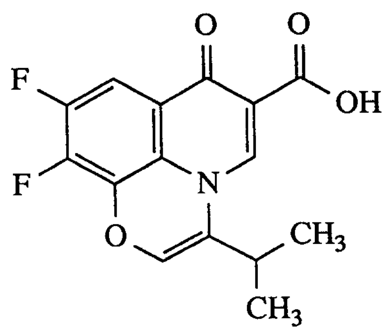

2,50 g (7,5 mmol) der Verbindung aus Beispiel XV werden in einem Gemisch aus 100 ml Wasser, 110 ml Eisessig und 10 ml Schwefelsäure 4 h unter Rückfluß erhitzt. Nach dem Abkühlen wird mit Wasser versetzt. Der ausgefallene Niederschlag wird abgesaugt und im Vakuum getrocknet.
Ausbeute: 1,76(77 %);
Schmp.: 253-255 ° C.

Beispiel XVIII

9,10-Difluor-3-ethyl-7H-7-oxopyrido[1,2,3-de][1,4]benzoxacin-6-carbonsäure

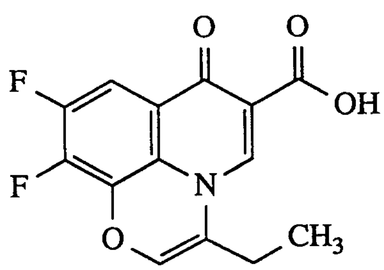

In Analogie zur Vorschrift des Beispiels XVII wird aus der Verbindung des Beispiels XVI die Titelverbindung hergestellt.
Ausbeute: 68 %;
Schmp.: 246 ° C.

Beispiel XIX

9,10-Difluor-3-methyl-7-oxo-7H-pyrido[1,2,3-de][1,4]benzoxacin-6-carbonsäure

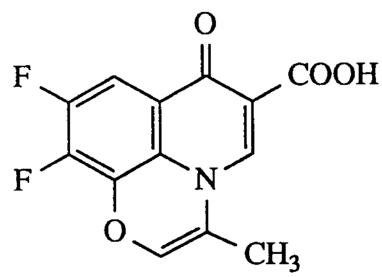

11,0 g 6,7,8-Trifluor-1,4-dihydro-4-oxo-1-(1-oxo-2-propyl)-3-chinolincarbonsäure werden mit 4,62 g KOtBut in 110 ml tert. Butanol 3 Stunden zum Sieden erhitzt. Nach Abkühlen auf Raumtemperatur wird auf Wasser gegeben, angesäuert und der ausfallende Feststoff isoliert.
Ausbeute: 8,5 g;
Schmp.: 258-260 °C.

Beispiel XX

10-(4-Ethoxycarbonyl-1-piperazinyl)-9-fluor-3-methyl-7-oxo-7H-pyrido[1,2,3-de][1,4]benzoxacin-6-carbonsäure

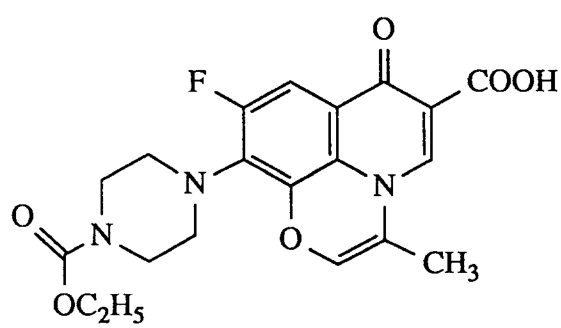

0,21 g 9,10-Difluor-3-methyl-7-oxo-7H-pyrido[1,2,3-de][1,4]benzoxacin-6-carbonsäure und 0,36 g Ethoxycarbonylpiperazin werden in 7,5 ml DMSO 3 Stunden auf 120 °C erwärmt. Alle flüchtigen Komponenten werden im Hochvakuum abdestilliert und der Rückstand mit Ethanol verrührt.
Ausbeute: 0,22 g;
Schmp.: 290-292 °C.

Beispiel XXI

9,10-Difluor-2-(4-methyl-phenyl)-7-oxo-7H-pyrido[1,2,3-de][1,4]benzoxacin-6-carbonsäureethylester

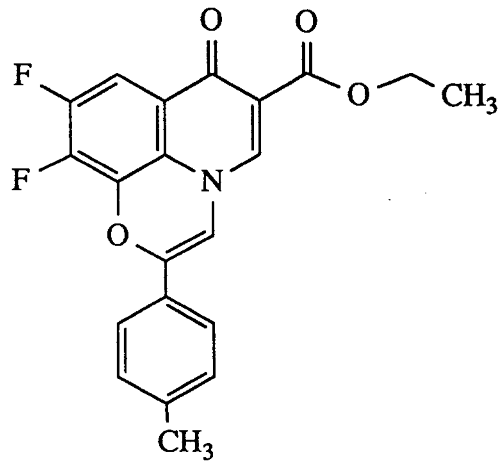

Analog zu Beispiel III erhält man mit 2-Brom-4'-methyl-acetophenon 0,52 g (52 % der Theorie) der Titelverbindung.
Schmp.: 237 ° C.

Beispiel XXII

9,10-Difluor-2-ethyl-7-oxo-7H-pyrido[1,2,3-de][1,4]benzoxacin-6-carbonsäureethylester

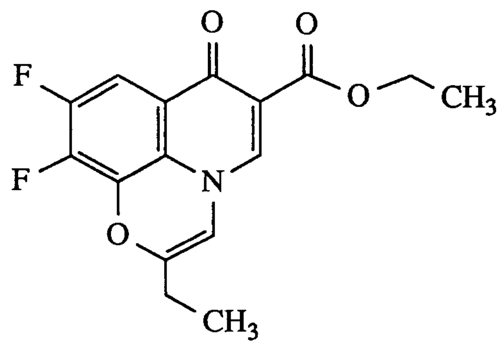

Analog zu Beispiel II erhält man mit 1-Brom-2-butanon 0,9 g (38 % der Theorie) der Titelverbindung.
Schmp: 210 ° C.

In Analogie zu den Vorschriften der Beispiele VI und VII werden die in Tabelle II aufgeführten Verbindungen hergestellt:

Tabelle II:

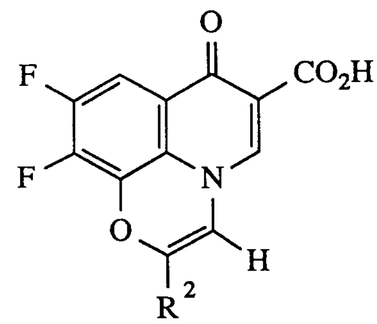

| Beispiel-Nr. | $R^2$ | F°C |
|---|---|---|
| XXIII | $-C_2H_5$ | >230 |
| XXIV | $-C_6H_4$-p-$CH_3$ | >230 |

Beispiel XXV

2-[1,1-Bis(fluormethyl)-1-ethyl]-9,10-difluor-7-oxo-7H-pyrido[1,2,3-de][1,4]benzoxacin-6-carbonsäureethylester

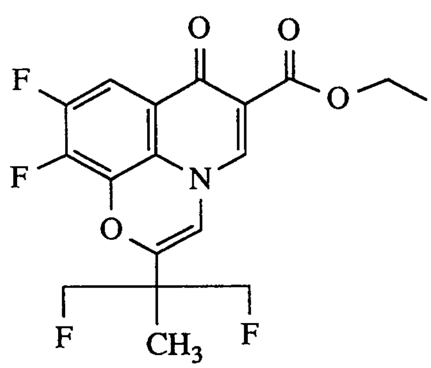

In Analogie zur Vorschrift von Beispiel III erhält man in 23 % der Theorie der Titelverbindung. Schmp.: 225 ° C.

Beispiel XXVI

2-[1,1-Bis(fluormethyl)-ethyl]-9,20-difluor-7-oxo-7H-pyrido[1,2,3-de][1,4]benzoxacin-6-carbonsäure

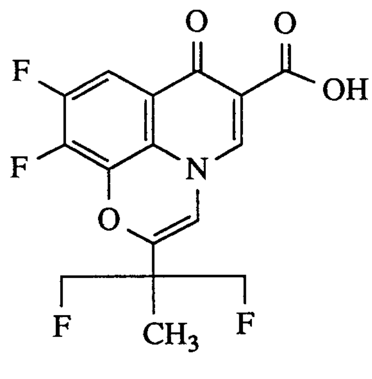

In Analogie zur Vorschrift von Beispiel VI erhält man in 85 % der Theorie der Titelverbindung.
Schmp.: >230 °C.

Beispiel XXVII

9,10-Difluor-7H-7-oxo-3-phenyl-pyrido[1,2,3-de] [1,4]benzoxazin-6-carbonsäureethylester

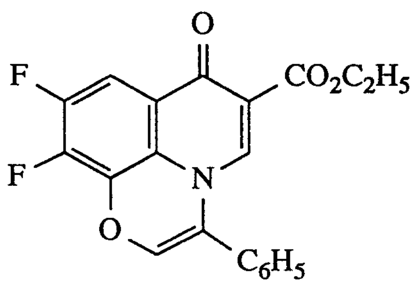

Die Titelverbindung wird in Analogie zur Vorschrift des Beispiels XV aus der Verbhindung des Beispiels XIII hergestellt.

Beispiel XXVIII

9,10-Difluor-7H-7-oxo-3-phenyl-pyrido[1,2,3-de][1,4]benzoxazin-6-carbonsäure

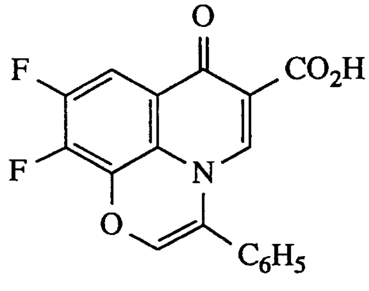

Die Titelverbindung wird in Analogie zur Vorschrift des Beispiels XVII aus der Verbindung des Beispiels XXVII hergestellt.

Beispiel XXIX

1-(2-Ethyl-1-hexen-3-yl)-4H-4-oxo-6,7,8-trifluorchinolin-3-carbonsäure-ethylester

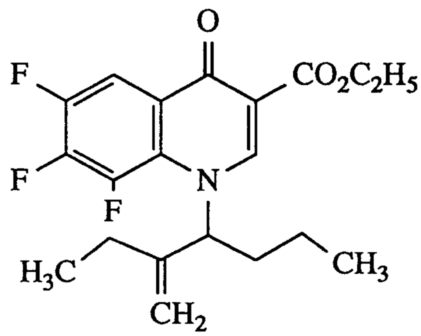

In Analogie zur Vorschrift des Beispiels XII wird die Titelverbindung aus 3-Amino-2-ethyl-1-hexen hergestellt.

Beispiel XXX

9,10-Difluor-2-ethyl-3-propyl-7H-7-oxo-pyrido-[1,2,3-de][1,4]benzoxazin-6-carbonsäure-ethylester

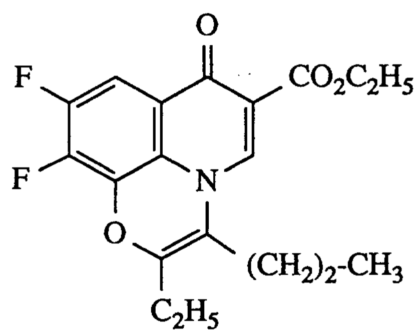

Die Titelverbindung wird in Analogie zur Vorschrift des Beispiels XV aus der Verbindung des Beispiels XXIX hergestellt.

Beispiel XXXI

1-(2-Methyl-1-buten-3-yl)-4H-7-oxo-6,7,8-trifluorchinolin-3-carbonsäure-ethylester

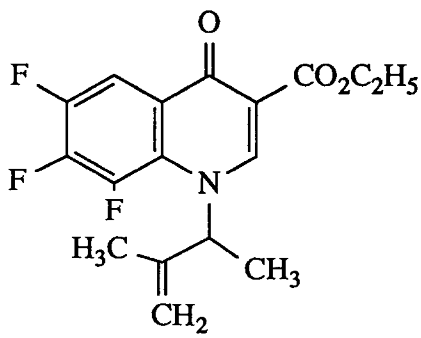

Die Titelverbindung wird in Analogie zur Vorschrift des Beispiels XII aus 3-Amino-2-methyl-1-buten hergestellt.
F°C: 122.

Beispiel XXXII

9,10-Difluor-7H-7-oxo-2-ethyl-3-(1-propyl)-pyrido[1,2,3-de][1,4]benzoxazin-6-carbonsäure

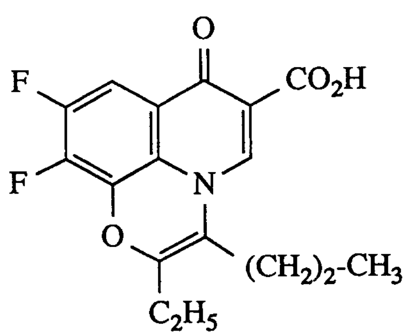

Die Titelverbindung wird in Analogie zur Vorschrift des Beispiels XVII aus der Verbindung des Beispiels XXX hergestellt.
F°C: 170.

Beispiel XXXIII

9,10-Difluor-2,3-dimethyl-7H-7-oxo-pyrido[1,2,3-de][1,4]benzoxazin-6-carbonsäure-ethylester

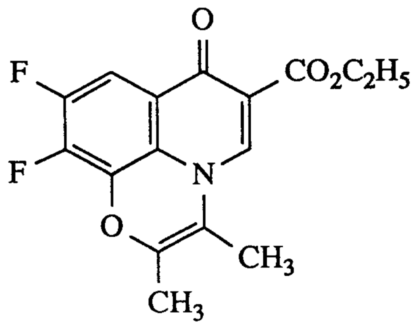

Die Titelverbindung wird in Analogie zur Vorschrift des Beispiels XV aus der Verbindung des Beispiels XXXI hergestellt.
F°C: 186.

Beispiel XXXIV

9,10-Difluor-2,3-dimethyl-7H-7-oxo-pyrido[1,2,3-de][1,4]benzoxazin-6-carbonsäure

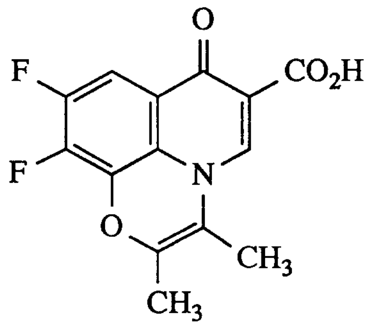

Die Titelverbindung wird in Analogie zur Vorschrift des Beispiels XVII aus der Verbindung des Beispiels XXXIII hergestellt.

Beispiel XXXV

1-(4,4-Dimethyl-1-penten-3-yl)-4H-4-oxo-6,7,8-trifluorchinolin-3-carbonsäure-ethylester

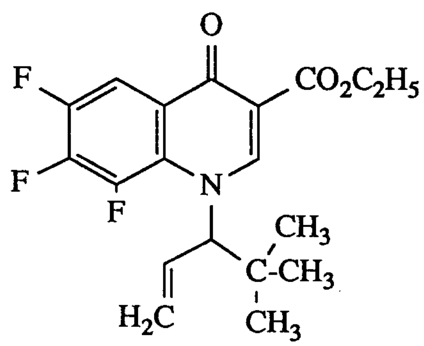

Die Titelverbindung wird in Analogie zur Vorschrift des Beispiels XII aus 3-Amino-4,4-dimethyl-1-penten hergestellt.

Beispiel XXXVI

9,10-Difluor-7H-7-oxo-3-(1,1-dimethyl-1-ethyl)-pyrido[1,2,3-de][1,4]benzoxazin-6-carbonsäureethylester

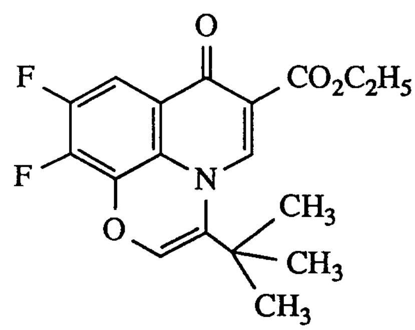

Die Titelverbindung wird in Analogie zur Vorschrift des Beispiels XV aus der Verbindung des Beispiels XXXV hergestellt.

Beispiel XXXVII

9,10-Difluor-7H-7-oxo-3-(1,1-dimethyl-1-ethyl)-pyrido[1,2,3-de][1,4]benzoxazin-6-carbonsäure

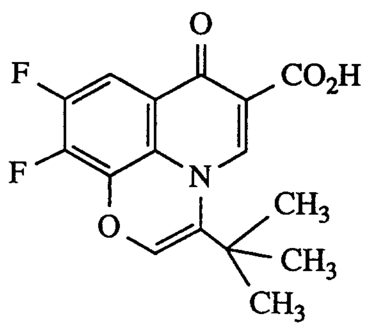

Die Titelverbindung wird in Analogie zur Vorschrift des Beispiels XVII aus der Verbindung des Beispiels XXXVI hergestellt.

Herstellungsbeispiele

Beispiel 1

2-tert-Butyl-9-fluor-10-(4-methyl-1-piperazinyl)-7-oxo-7H-pyrido[1,2,3-de][1,4]-benzoxacin-6-carbonsäure

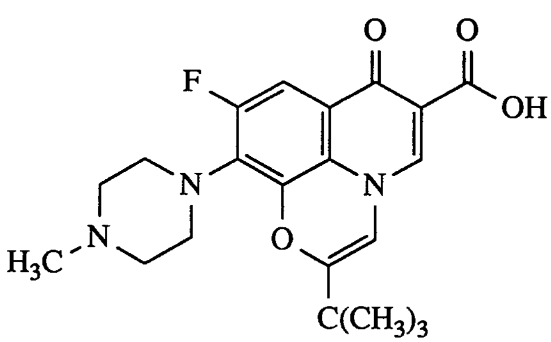

100 mg (0,31 mmol) der Verbindung aus Beispiel VI, 80 mg (0,62 mmol) Diisopropyl-ethyl-amin und 93 mg (0,93 mmol) N-Methylpiperazin werden in 2 ml abs. DMSO unter Argon 3 h auf 140 °C erhitzt. Nach Abkühlen wird mit Wasser versetzt und der Feststoff verrührt, dann filtriert. Das Rohprodukt wird mit Wasser gewaschen und im Hochvakuum getrocknet.
Ausbeute: 88 mg (70% der Theorie)
Schmp.: >230 °C

Beispiel 2

9-Fluor-7-oxo-10-(1-piperazinyl)-7H-pyrido[1,2,3-de][1,4]benzoxacin-6-carbonsäure

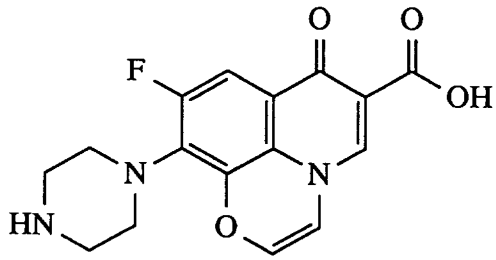

130 mg (0,30 mmol) der Verbindung aus Beispiel XI werden bei Raumtemperatur 1 h mit einem Gemisch aus 1 ml Anisol und 10 ml Trifluoressigsäure behandelt. Man engt im Vakuum ein, versetzt den Rückstand mit Ether und saugt ab. Man löst den Feststoff in Wasser, stellt mit Natriumacetat auf pH 5 und chromatographiert an RP-18 mit Wasser / Acetonitril / Essigsäure.
Ausbeute: 35 mg (35% der Theorie)
$^{1}$H-NMR ($CF_3CO_2D$): $\delta$ = 7,02 (s; 2H, 2-H und 3-H); 7,88 (d, J = 11 Hz; 1H, 8-H); 8,37 (s; 1H, 5-H).

Beispiel 3

9-Fluor-10-(3-methyl-1-piperazinyl)-7-oxo-7H-pyrido[1,2,3-de][1,4]benzoxacin-6-carbonsäure

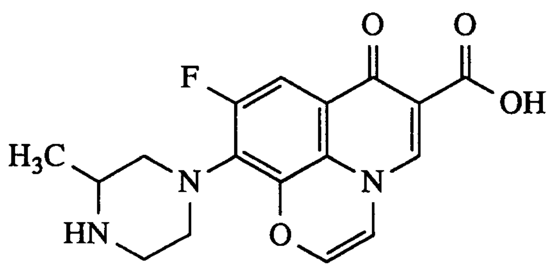

1,0 g (3,5 mmol) 1-Formylmethyl-4-oxo-6,7,8-trifluor-4H-chinolin-3-carbonsäure und 0,7 g (7,0 mmol) 2-Methylpiperazin werden in einem Gemisch aus 5 ml DMSO und 6 ml Disopropyl-ethylamin 4 h unter Rückfluß erhitzt. Nach dem Abkühlen versetzt man mit Ether, dekantiert das Lösemittel ab und verreibt den Rückstand mit Essigester und Ethanol. Zur Reinigung löst man den Rückstand in Wasser und chromatographiert an RP-18 mit Wasser / Acetonitril / Essigsäure.
Ausbeute: 25 mg (2% der Theorie)
$^{1}$H-NMR (DMSO-$d_6$): $\delta$ = 0,96 (d, 1 = 6 Hz; 3H, $CH_3$); 6,94 und 7,08 (2d, J = 5 Hz; je 1 H, 2-H und 3-H); 7,42 (d, J = 12,5 Hz; 1H, 8-H); 8,72 (s; 1H, 5-H).

Beispiel 4

10-(3,5-Dimethyl-1-piperazinyl)-9-fluor-7-oxo-7H-pyrido[1,2,3-de][1,4]benzoxacin-6-carbonsäure

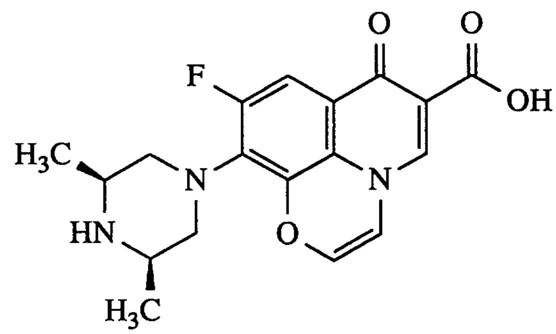

Analog zu Beispiel 3 wurde die Titelverbindung hergestellt.
Ausbeute: 15 %
$^1$H-NMR (DMSO-$d_6$): δ = 1,30 (d, J = 6 Hz; 3H, $CH_3$); 6,98 und 7,13 (2d, J = 5 Hz; je 1H, 2-H und 3-H); 7,48 (d, J = 13 H;, 1H, 8-H); 8,76 (s; 1H, 5-H).

Beispiel 5

9-Fluor-3-methyl-10-(4-ethyl-1-piperazinyl)-7-oxo-7H-pyrido[1,2,3-de][1,4]benzoxacin-6-carbonsäure

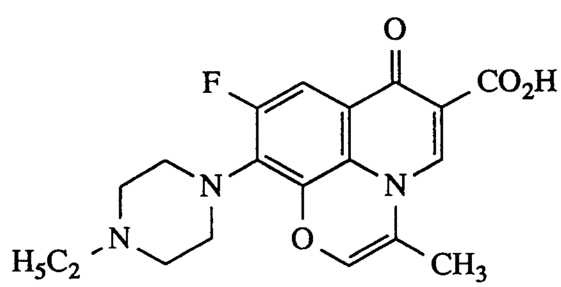

300 mg (1,075 mmol) 9,10-Difluor-3-methyl-7-oxo-7H-pyrido[1,2,3-de][1,4]benzoxacin-6-carbonsäure werden in 8 ml DMSO suspendiert, mit 139,8 mg (1,075 mmol) 1-Ethyl-piperazin und 240,8 mg (2,15 mmol) DABCO versetzt und 1 h bei 100 °C erhitzt. Nach vollständiger Umsetzung wird das Lösemittel im Hochvakuum abdestilliert, der Rückstand mit Isopropanol aufgeschlämmt und der Feststoff abgesaugt und getrocknet.
Ausbeute: 354 mg (85% der Theorie) als Feststoff
In Analogie zu den Vorschriften der Beispiele 1-5 werden die in den Tabellen 1, 2, 3, 4, 5 und 6 aufgeführten Verbindungen hergestellt; wobei im Fall der Ester ($R^4$ ≠ H) die Ester der entsprechenden Vorstufen direkt umgesetzt werden:

Tabelle 1:

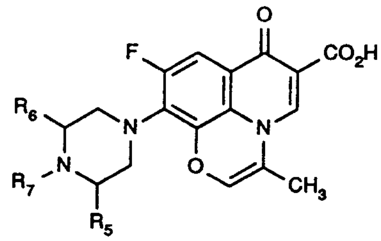

| Bsp.-Nr. | $R^5$ | $R^6$ | $R^7$ | F°C | Ausbeute (% d.Th.) |
|---|---|---|---|---|---|
| 6 | H | H | $-CH_2-C_6H_5$ | 237 | 82 |
| 7 | H | H | $-(CH_2)_2-OH$ | 183 | 83 |
| 8 | H | H | $H_3C-CH(OH)-CH_2-$ | 238 | 64 |
| 9 | H | H | $-CH(C_6H_5)-CH_3$ | 210 | 50 |
| 10 | H | $-C_6H_5$ | H | 242 | 37 |
| 11 | H | $-C_6H_{11}$ | H | 195 | 53 |
| 12 | $-H_3C$ | $-H_3C$ | H | >250 | 73 |
| 13 | H | $-C_2H_5$ | H | 170 | 39 |
| 14 | H | $-C(CH_3)_3$ | H | 145 | 31 |

Tabelle 2:

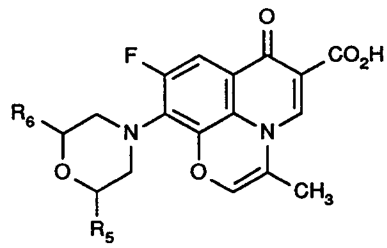

| Bsp.-Nr. | $R^5$ | $R^6$ | F°C | Ausbeute (% d.Th.) |
|---|---|---|---|---|
| 15 | $-CH_3$ | $-CH_3$ | >251 | 51 |
| 16 | H | H | >265 | 83 |

Tabelle 3:

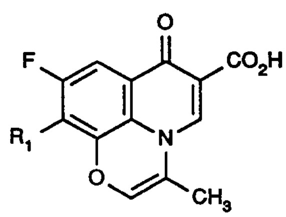

| Bsp.-Nr. | $R^1$ | F°C | Ausbeute (% d.Th.) |
|---|---|---|---|
| 17 | 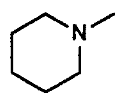 | 247 | 31 |

Tabelle 4:

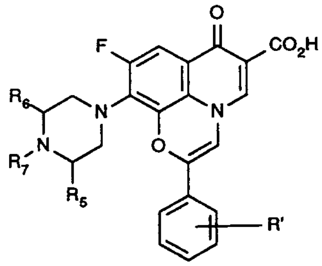

| Bsp.-Nr. | $R^5$ | $R^6$ | $R^7$ | R' | F°C | Ausbeute (% d.Th.) |
|---|---|---|---|---|---|---|
| 18 | H | H | $-CH_3$ | $o-OCH_3$ | >230 | 23 |
| 19 | H | H | H | $o-OCH_3$ | >230 | 25 |
| 20 | $-CH_3$ | $-CH_3$ | H | $o-OCH_3$ | >230 | 96 |
| 21 | H | H | $-CH_3$ | H | | |
| 22 | H | H | H | H | | |
| 23 | $-CH_3$ | H | H | $m-CH_3$ | >230 | 41 |

Tabelle 5:

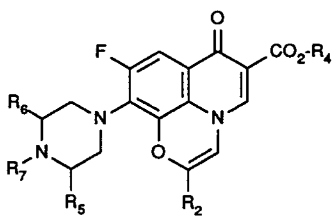

| Bsp.-Nr. | $R^2$ | $R^4$ | $R^5$ | $R^6$ | $R^7$ | in Analogie zu Beispiel | F°C | Ausbeute (% d.Th.) |
|---|---|---|---|---|---|---|---|---|
| 24 | $-C(CH_3)_3$ | $-C_2H_5$ | $-CH_3$ | H | H | 1 | 110 | 70 |
| 25 | $-C(CH_3)_3$ | $-C_2H_5$ | H | H | $-CH_3$ | 1 | 160 | 57 |
| 26 | $-CH(CH_3)_2$ | $-C_2H_5$ | H | H | $-CH_3$ | 1 | 103 | 55 |
| 27 | $-CH(CH_3)_2$ | H | H | H | $-CH_3$ | 1 | >230 | 50 |
| 28 | $-CH(CH_3)_2$ | H | $-CH_3$ | H | H | 1 | >230 | 17 |
| 29 | $-CH(CH_3)_2$ | H | $-CH_3$ | $-CH_3$ | H | 1 | >230 | 25 |

Tabelle 6:

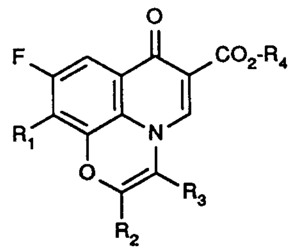

| Bsp.-Nr. | $R^1$ | $R^2$ | $R^3$ | $R^4$ | F°C |
|---|---|---|---|---|---|
| 30 | 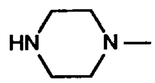 | $-C_6H_4$-p-$OCH_3$ | H | $-C_2H_5$ | |
| 31 | 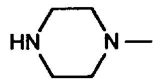 | $-C_6H_4$-p-Cl | H | $-C_2H_5$ | |
| 32 | 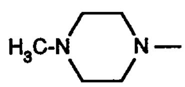 | $-C_6H_4$-p-Cl | H | $-C_2H_5$ | |
| 33 | 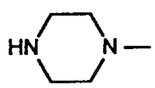 | H | $-CH_3$ | $-CH_3$ | 280 (Z.) |

Beispiel 34

2-tert.-Butyl-9-fluor-10-(3,5-dimethyl-1-piperazinyl)-7-oxo-7H-pyrido[1,2,3-de][1,4]benzoxacin-6-carbonsäure

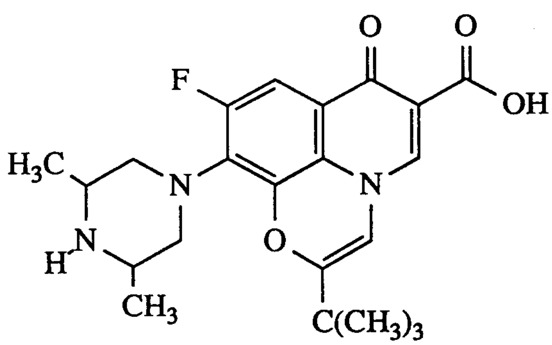

Analog zu Beispiel 1 erhält man mit 2,6-Dimethyl-piperazin 96 mg (54 % der Theorie) der Titelverbindung. Schmp.: >230 ° C.

Beispiel 35

2-tert.-Butyl-9-fluor-10-(3-methyl-1-piperazinyl)-7-oxo-7H-pyrido[1,2,3-de][1,4]benzoxacin-6-carbonsäure

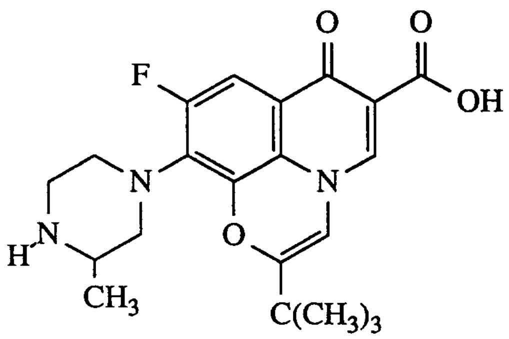

Analog zu Beispiel 1 erhält man mit 2-Methyl-piperazin 72 mg (58 % der Theorie) der Titelverbindung. Schmp.: >230 °C.

Beispiel 36

2-tert.-Butyl-9-fluor-10-(1-piperazinyl)-7-oxo-7H-pyrido[1,2,3-de][1,4]benzoxacin-6-carbonsäure

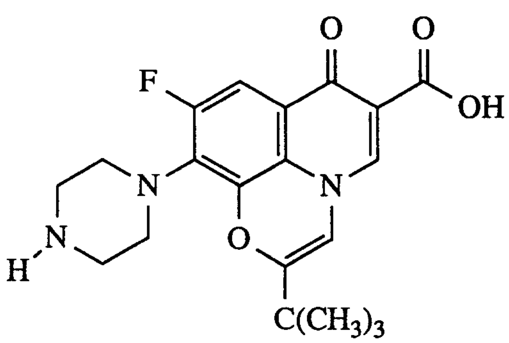

Analog zu Beispiel 1 erhält man mit Piperazin 90 mg (37 % der Theorie) der Titelverbindung. Schmp.: >230 °C.

Beispiel 37

10-(3-Amino-1-pyrrolidinyl)-2-(4-chlorphenyl)-9-fluor-7-oxo-7H-pyrido[1,2,3-de][1,4]benzoxacin-6-carbonsäureethylester-hydrochlorid

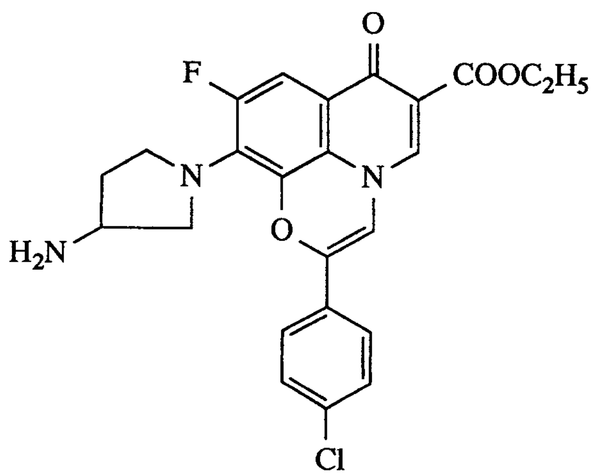

x HCl

0,4 g (1 mmol) 2-(4-Chlorphenyl)-9, 10-difluor-7-oxo-7H-pyrido[1,2,3-de][1,4]benzoxacin-6-carbonsäureethylester, 0,31 g (1,6 mmol) 3-tert.Butoxycarbonylaminopyrrolidin und 0,22 g (2 mmol) 1,4-Diazabicyclo[2,2,2]-octan werden in 10 ml N-Methylpyrrolidon 2 h auf 140 ° C erwärmt. Es wird mit Wasser versetzt und isoliert. Die Reinigung des Rohproduktes erfolgt durch Säulenchromatographie. Anschließend werden 0,4 g des Produktes 2 h in einer Mischung aus 3,7 ml Methanol und 3,7 mi konz. Salzsäure gerührt. Die Lösemittel werden im Vakuum abdestilliert und der Rückstand mit Isopropanol verrührt.
Ausbeute: 0,23 g
Schmp.: 271 ° C (Zers.)

Beispiel 38

10-(3-Amino-1-pyrrolidinyl)-9-fluor-2-(4-methoxyphenyl)-7-oxo-7H-pyrido[1,2,3-de][1,4]benzoxacin-6-carbonsäureethylester-hydrochlorid

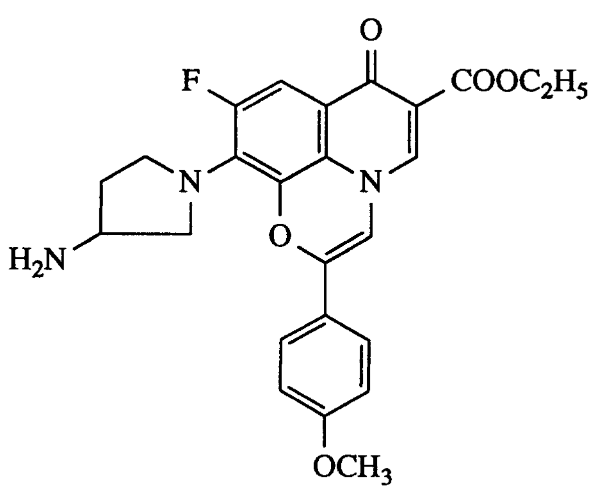

x HCl

Analog zu Beispiel 37 kann die Titelverbindung ausgehend von 9,10-Difluor-2-(4-methoxyphenyl)-7-oxo-7H-pyrido[1,2,3-de][1,4]benzoxacin-6-carbonsäureethylester erhalten werden.
Schmp.: 250 ° C (Zers.)

In Analogie zu den Vorschriften der Beispiele 5, 37 und 38 werden die in Tabelle 7 aufgeführten Verbindungen hergestellt:

Tabelle 7:

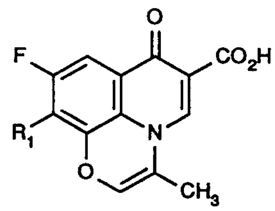

| Bsp.-Nr. | $R^1$ | Salz | F°C |
|---|---|---|---|
| 39 | 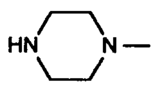 | HCl | >300 |

Die in Tabelle 8 aufgeführten Verbindungen werden in Analogie zu den dort angegebenen Beispielen hergestellt:

Tabelle 8:

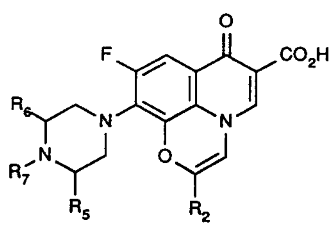

| Bsp.-Nr. | $R^2$ | $R^5$ | $R^6$ | $R^7$ | in Analogie zu Beispiel | F°C | Ausbeute (% d.Th.) |
|---|---|---|---|---|---|---|---|
| 40 | $-CH(CH_3)_2$ | H | H | H | 1 | >230 | 44 |
| 41 | $-C_2H_5$ | H | H | H | 1 | >230 | 43 |
| 42 | $-C_2H_5$ | $-CH_3$ | H | H | 1 | >230 | 47 |
| 43 | $-C_2H_5$ | $-CH_3$ | $-CH_3$ | H | 1 | >230 | 75 |
| 44 | $-C_2H_5$ | H | H | $-CH_3$ | 1 | >230 | 35 |

Beispiel 45

9-Fluor-7H-7-oxo-10-(1-piperazinyl)-3-(2-propyl)-pyrido[1,2,3-de][1,4]benzoxacin-6-carbonsäure

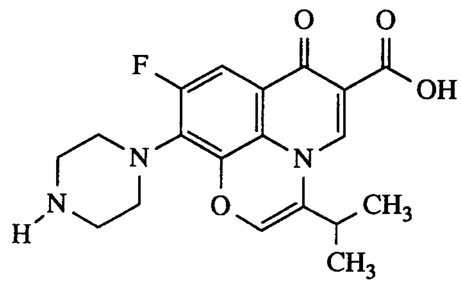

307 mg (1,0 mmol) der Verbindung aus Beispiel XVII, 5 ml DMSO, 1,7 ml Diisopropylethylamin und 172 mg (2,0 mmol) Piperazin werden 4 h unter Rückfluß erhitzt. Nach dem Abkühlen fügt man 50 ml Ether zu, saugt ab, wäscht den Niederschlag mit Ether und trocknet im Vakuum.
Ausbeute: 298 mg; (80 %).

Beispiel 46

3-Ethyl-9-fluor-10-(4-methyl-1-piperazinyl)-7H-7-oxo-pyrido[1,2,3-de][1,4]benzoxacin-6-carbonsäure

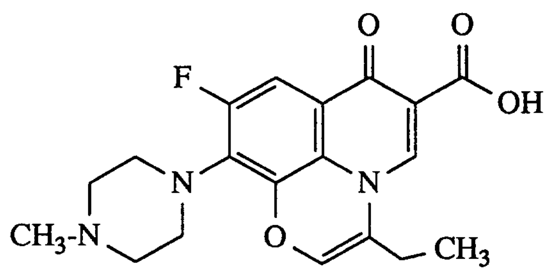

Die Titelverbindung wird in Analogie zur Vorschrift des Beispiels 45 hergestellt.
Ausbeute: 23 % der Theorie.

Beispiel 47

3-Ethyl-9-fluor-10-(N-morpholino)-7H-7-oxo-pyrido[1,2,3-de][1,4]benzoxacin-6-carbonsäure

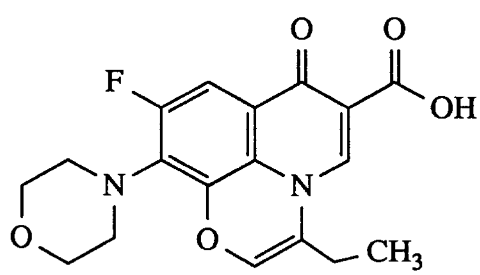

In Analogie zur Vorschrift des Beispiels 45 wird die Titelverbindung hergestellt.
Ausbeute: 25 % der Theorie.

In Analogie zu der Vorschrift des Beispiels 45 werden die in Tabelle 9 aufgeführten Verbindungen hergestellt:

Tabelle 9:

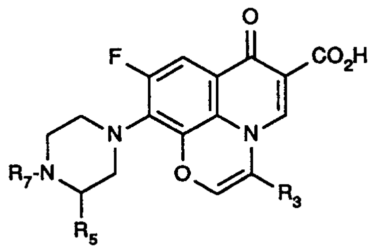

| Bsp.-Nr. | $R^3$ | $R^5$ | $R^7$ | Ausbeute (% d.Th.) |
|---|---|---|---|---|
| 48 | $-CH(CH_3)_2$ | $-CH_3$ | H | 55 |
| 49 | $-C_2H_5$ | H | H | 58 |
| 50 | $-C_2H_5$ | $-CH_3$ | H | 43 |

Zu den Verbindungen liegen $^1$H-NMR-Daten vor.

In Analogie zur Vorschrift des Beispiels 1 werden die in Tabelle 10 aufgeführten Verbindungen hergestellt:

Tabelle 10:

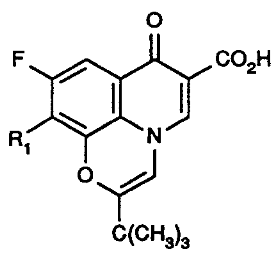

| Bsp.-Nr. | $R^1$ | F°C |
|---|---|---|
| 51 | 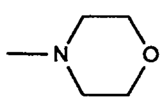 | >250 |
| 52 | 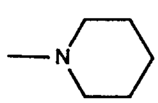 | >250 |

Beispiel 53

2-[1,1-Bis(fluormethyl)-1-ethyl]-10-(3,5-dimethyl-1-piperazinyl)-9-fluor-7-oxo-7H-pyrido[1,2,3-de][1,4]-benzoxacin-6-carbonsäurehydrochlorid

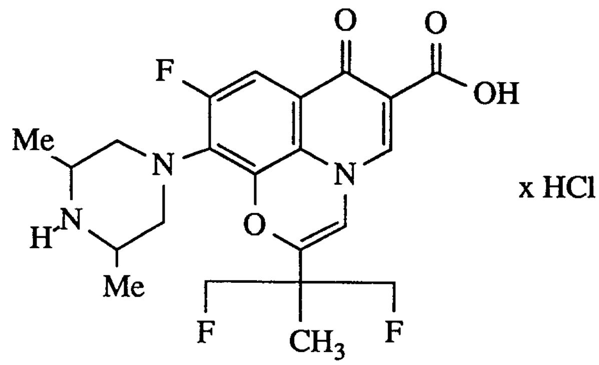

200 mg (0,56 mmol) der Verbindung aus Beispiel XXVI, 145 mg (1,12 mmol) Diisopropylethylamin und 192 mg (1,68 mmol) 2,6-Dimethylpiperazin werden in 2 ml abs. DMSO unter Argon 4 Stunden auf 140 °C erhitzt. Man läßt abkühlen und versetzt mit 15 ml Ether. Der Niederschlag wird abgesaugt und mit Ether gewaschen. Man löst den Feststoff in 1,5 ml konz. HCl, addiert 75 ml Ethanol und läßt über Nacht rühren. Das Produkt wird abgesaugt und im Hochvakuum getrocknet. Man erhält 51 mg (19 % der Theorie) der Titelverbindung. Schmelzpunkt: >230 °C.

Beispiel 54

2-[1,1-Bis(fluormethyl)-1-ethyl]-9-fluor-10-(4-methyl-1-piperazinyl)-7-oxo-7H-pyrido[1,2,3-de][1,4]benzoxacin-6-carbonsäure-hydrochlorid

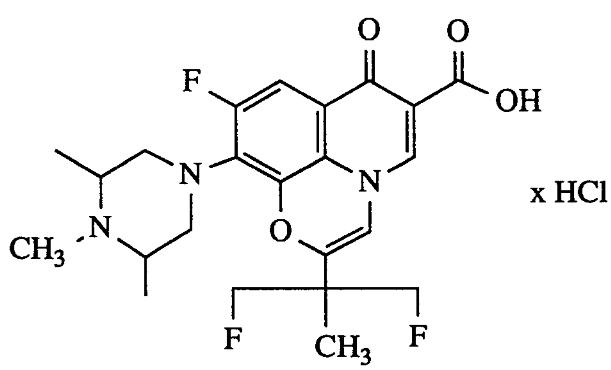

In Analogie zur Vorschrift des Beispiels 53 erhält man 89 mg (34 % der Theorie) der Titelverbindung. Schmelzpunkt: >230 °C.

Beispiel 55

9-Fluor-2-methyl- 10-(3-methyl-1-piperazinyl)-7-oxo-7H-pyrido[1,2,3-de][1,4]benzoxacin-6-carbonsäure

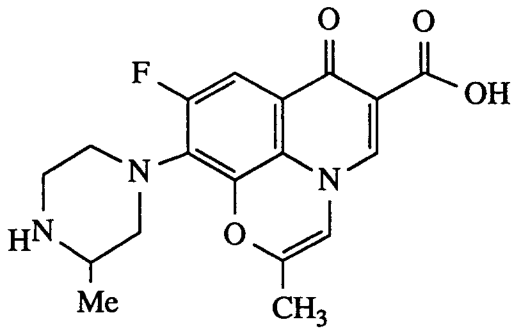

200 mg (0,72 mmol) des analog zu Beispiel 13 EP 253 235 hergestellte Startmaterials werden mit 174 mg (1,43 mmol) Hünigbase und 215 mg (2,15 mmol) 2-Methylpiperazin in 6 ml DMSO suspendiert und 5 Stunden unter Argon auf 140 °C erhitzt. Nach Abkühlen wird das DMSO im Vakuum entfernt. Man versetzt den Rückstand mit etwas Wasser und stellt auf pH 3 ein. Der ausgefallene Feststoff wird abgesaugt und im Vakuum getrocknet. Man erhält 97 mg (38 % der Theorie) der Titelverbindung.
Schmelzpunkt: >230 °C.

Analog zur Vorschrift des Beispiels 1 erhält man die in der Tabelle 11 aufgeführten Verbindungen.

Tabelle 11:

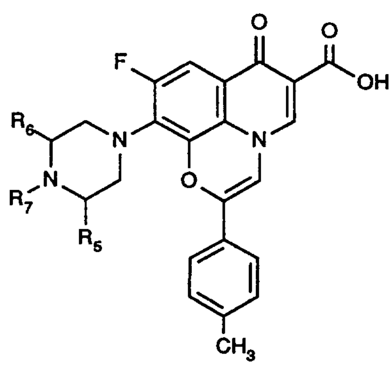

| Bsp.-Nr. | $R^5$ | $R^6$ | $R^7$ | F°C | Ausbeute (% d.Th.) |
|---|---|---|---|---|---|
| 56 | $-CH_3$ | $-CH_3$ | H | >230 | 64 |
| 57 | $-CH_3$ | H | H | >230 | 50 |

In Analogie zur Vorschrift des Beispiels 45 werden die in Tabelle 12 aufgeführten Verbindungen hergestellt:

Tabelle 12:

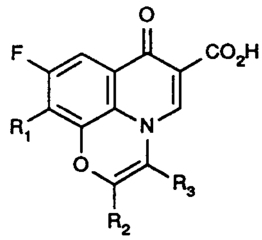

| Bsp.-Nr. | $R^1$ | $R^2$ | $R^3$ | F°C | Ausgangsverbindung |
|---|---|---|---|---|---|
| 58 | 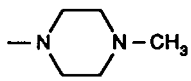 | H | $-CH(CH_3)_2$ | >250 | XVII |
| 59 | 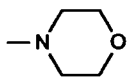 | H | $-CH(CH_3)_2$ | 230 | XVII |
| 60 | 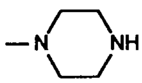 | H | $-C_6H_5$ | | XXVIII |
| 61 | 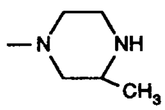 | H | $-C_6H_5$ | | XXVIII |
| 62 | 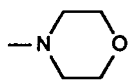 | H | $-C_6H_5$ | | XXVIII |
| 63 | 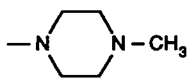 | H | $-C_6H_5$ | | XXVIII |
| 64 | 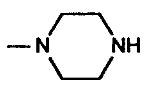 | $-C_2H_5$ | $-(CH_2)_2-CH_3$ | >250 | XXXII |
| 65 | 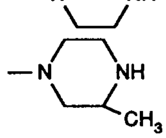 | $-C_2H_5$ | $-(CH_2)_2-CH_3$ | | XXXII |
| 66 | 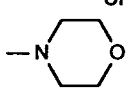 | $-C_2H_5$ | $-(CH_2)_2-CH_3$ | | XXXII |
| 67 | 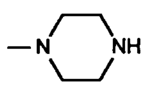 | $-CH_3$ | $-CH_3$ | | XXXIV |
| 68 | 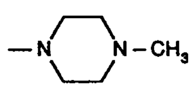 | $-CH_3$ | $-CH_3$ | | XXXIV |

Tabelle 12: (Fortsetzung)

| Bsp.-Nr. | $R^1$ | $R^2$ | $R^3$ | F°C | Ausgangsverbindung |
|---|---|---|---|---|---|
| 69 | 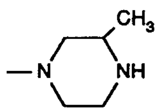 | $-CH_3$ | $-CH_3$ | | XXXIV |
| 70 | 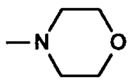 | $-CH_3$ | $-CH_3$ | | XXXIV |
| 71 | 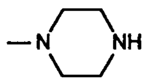 | H | $-C(CH_3)_3$ | | XXXVII |
| 72 | 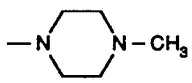 | H | $-C(CH_3)_3$ | | XXXVII |

In Analogie zur Vorschrift des Beispiels 5 werden die in Tabelle 13 aufgeführten Verbindungen hergestellt:

Tabelle 13:

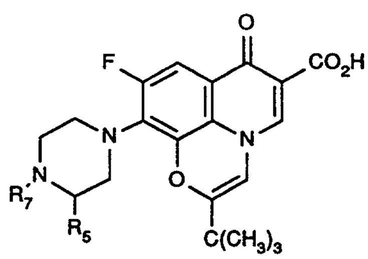

| Bsp.-Nr. | $R^7$ | $R^5$ | F°C |
|---|---|---|---|
| 73 | $-CH_2C_6H_5$ | H | >250 |
| 74 | $-CH-CH_3$<br>$C_6H_5$ | H | >250 |
| 75 | $-CH_2\cdot CH_2$<br>OH | H | |
| 76 | H | $C_2H_5$ | >250 |
| 77 | $-CH_2CH-CH_3$<br>OH | H | >250 |

Beispiel 78

2-tert.-Butyl-9-fluor-10-(4-methyl-1-piperazinyl)-7-oxo-7H-pyrido[1,2,3-de][1,4]benzoxacin-6-carbonsäure-hydrochlorid

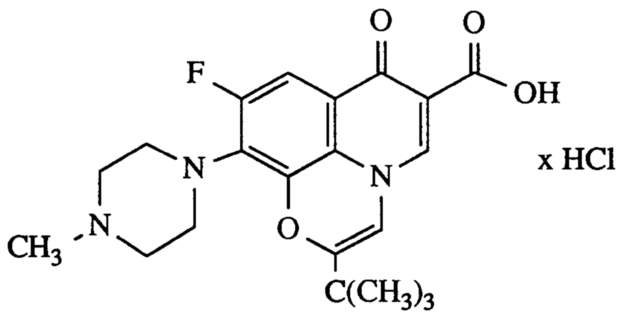

5,5 g(13,71 mmol) der Verbindung aus Beispiel 1 werden in 57 ml konz. HCl gelöst. Man gibt 1,6 l Ethanol zu und läßt über Nacht rühren. Das ausgefallene Produkt wird abgesaugt und im Hochvakuum getrocknet.
Ausbeute: 4,2g (70 % der Theorie)
Schmelzpunkt: >230 °C.

**Patentansprüche**

**1.** 7-Oxo-7H-pyrido[1,2,3-de][1,4]benzoxacin-6-carbonsäure-Derivate der allgemeinen Formel

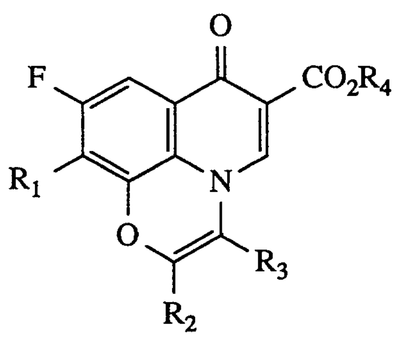

in welcher
$R^1$ für Halogen, 1-Piperidinyl oder für einen Rest der Formel

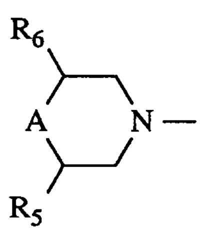

oder

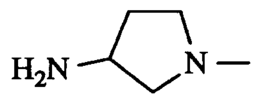

steht,

worin

A eine Gruppe der Formel $-NR^7$, ein Sauerstoffatom oder die $CH_2$-Gruppe bedeutet, worin

$R^7$ Wasserstoff oder geradkettiges oder verzweigtes Alkyl mit bis zu 6 Kohlenstoffatomen bedeutet, das gegebenenfalls durch Hydroxy oder Phenyl substituiert ist,

$R^5$ und $R^6$ gleich oder verschieden sind und Wasserstoff, Cyclohexyl, Phenyl oder geradkettiges oder verzweigtes Alkyl mit bis zu 4 Kohlenstoffatomen bedeuten,

$R^2$ für Wasserstoff, oder

für geradkettiges oder verzweigtes Perfluoralkyl mit bis zu 4 Kohlenstoffatomen steht, oder

für geradkettiges oder verzweigtes Alkyl mit bis zu 6 Kohlenstoffatomen steht, das gegebenenfalls durch Phenyl, Hydroxy, Halogen, geradkettiges oder verzweigtes Alkoxy oder Alkylthio mit jeweils bis zu 3 Kohlenstoffatomen substituiert ist,

für Phenyl, Pyridyl, Furyl oder Thienyl steht, die gegebenenfalls einfach durch Halogen oder durch geradkettiges oder verzweigtes Alkyl oder Alkoxy mit jeweils bis zu 3 Kohlenstoffatomen substituiert sind,

$R^3$ für Wasserstoff, Phenyl, Trifluorethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen steht,

in racemischer Form oder als optische Isomeren sowie deren Hydrate und Salze, mit Ausnahme von 9-Fluor-3-methyl-10(1-piperazinyl)-7-oxo-7H-pyrido[1,2,3-de][1,4]benzoxacin-6-carbonsäure und deren 3- und 4-Methyl-1-piperazinyl-Derivaten, 9-Fluor-10-(4-methyl-1-piperazinyl)-7-oxo-7H-pyrido[1,2,3-de]-[1,4]benzoxacin-6-carbonsäure und deren 2-Methyl-Derivat.

**2.** 7-Oxo-7H-pyrido[1,2,3-de][1,4]benzoxacin-6-carbonsäure-Derivate gemäß Anspruch 1, in welchen

$R^1$ für Fluor, 1-Piperidinyl oder für einen Rest der Formel

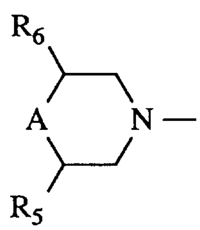

oder

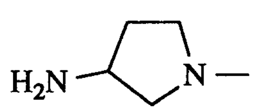

steht, worin

A eine Gruppe der Formel $-NR^7$, ein Sauerstoffatom oder die $CH_2$-Gruppe bedeutet, worin

$R^7$ Wasserstoff oder geradkettiges oder verzweigtes Alkyl mit bis zu 4 Kohlenstoffatomen

bedeutet, das gegebenenfalls durch Hydroxy oder Phenyl substituiert ist,

$R^5$ und $R^6$ gleich oder verschieden sind und Wasserstoff, Cyclohexyl, Phenyl oder geradkettiges oder verzweigtes Alkyl mit bis zu 4 Kohlenstoffatomen bedeuten,

$R^2$ für Wasserstoff oder
für geradkettiges oder verzweigtes Perfluoralkyl mit bis zu 4 Kohlenstoffatomen steht,
für geradkettiges oder verzweigtes Alkyl mit bis zu 5 Kohlenstoffatomen steht, das gegebenenfalls durch Phenyl, Hydroxy, Fluor, Chlor oder Alkoxy oder Alkylthio mit jeweils bis zu 2 Kohlenstoffatomen substituiert ist,
für Phenyl, Pyridyl, Furyl oder Thienyl steht, die gegebenenfalls einfach durch Fluor, Chlor, Brom oder geradkettiges oder verzweigtes Alkyl oder Alkoxy mit jeweils bis zu 3 Kohlenstoffatomen substituiert sind,

$R^3$ für Wasserstoff, Phenyl, Trifluormethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen steht.

**3.** 7-Oxo-7H-pyrido[1,2,3-de][1,4]benzoxacin-6-carbonsäure-Derivate gemaß Anspruch 1, in welcher

$R^1$ für 1-Piperidinyl steht, oder
für einen Rest der Formel

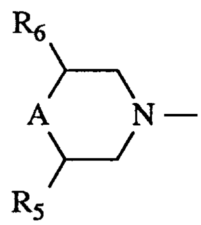

oder

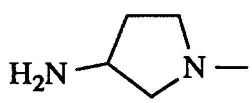

steht, worin

A eine Gruppe der Formel $-NR^7$, ein Sauerstoffatom oder die $CH_2$-Gruppe bedeutet, worin

$R^7$ Wasserstoff oder geradkettiges oder verzweigtes Alkyl mit bis zu 4 Kohlenstoffatomen bedeutet, das gegebenenfalls durch Hydroxy oder Phenyl substituiert ist,

$R^5$ und $R^6$ gleich oder verschieden sind und Wasserstoff, Cyclohexyl, Phenyl, oder geradkettiges oder verzweites Alkyl mit bis zu 4 Kohlenstoffatomen bedeuten,

$R^2$ für Wasserstoff oder
für geradkettiges oder verzweigtes Perfluoralkyl mit bis zu 4 Kohlenstoffatomen steht,
für geradkettiges oder verzweigtes Alkyl oder Alkenyl mit jeweils bis zu 5 Kohlenstoffatomen steht, das gegebenenfalls durch Phenyl, Hydroxy, Fluor, Chlor, Methoxy, Ethoxy oder Methylthio substituiert ist,
für Phenyl, Pyridyl oder Furyl steht, die gegebenenfalls einfach durch Fluor, Chlor, Brom, Methyl, Ethyl, Methoxy oder Ethoxy substituiert sind,

$R^3$ für Wasserstoff, Phenyl, Trifluormethyl, geradkettiges oder verzweigtes Alkyl mit bis zu 4 Kohlenstoffatomen, Fluormethyl oder Hydroxymethyl steht,

$R^4$ für Wasserstoff oder für geradkettiges oder verzweigtes Alkyl mit bis zu 4 Kohlenstoffatomen steht.

**4.** Verfahren zur Herstellung von 7-Oxo-7H-pyrido[1,2,3-de][1,4]benzoxacin-6-carbonsäure-Derivaten gemäß Anspruch 1, dadurch gekennzeichnet, daß man

[A] Verbindungen der allgemeinen Formel (II)

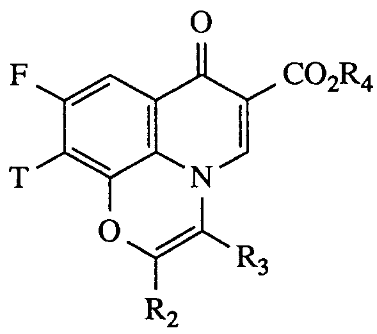

(II),

in welcher

$R^2$, $R^3$ und $R^4$ die oben angegebene Bedeutung haben,

und

T für Halogen steht,

mit Verbindungen der allgemeinen Formel (III)

$R^1$-H (III),

in welcher

$R^1$ die oben angegebene Bedeutung hat,

in inerten Lösemitteln, in Anwesenheit einer Base unter Schutzgasatmosphäre umsetzt, oder

[B] im Fall, daß $R^2$ und $R^3$ für Wasserstoff stehen,

auch Aldehyde der allgemeinen Formel (IV)

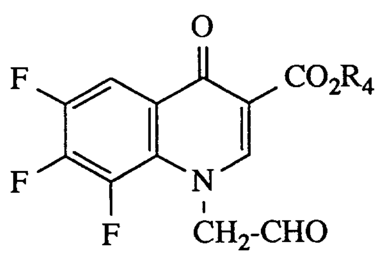

(IV),

in welcher

$R^4$ die oben angegebene Bedeutung hat,

mit Verbindungen der allgemeinen Formeln (III) oder (IIIa)

$R^1$-H (III),

W-H (IIIa),

in welcher

$R^1$ die oben angegebene Bedeutung hat

und

W die oben angegebene Bedeutung von $R^1$ hat, wobei aber bis auf eine die Aminfunktionen durch Schutzgruppen geschützt sind,

in inerten Lösemitteln, in Anwesenheit einer Base, unter gleichzeitiger Cyclisierung umsetzt, die Schutzgruppe nach üblicher Methode abspaltet und anschließend je nach gewünschter Bedeutung von $R^4$ entweder eine Veresterung oder Verseifung nach üblicher Methode durchführt.

**5.** 7-Oxo-7H-pyrido[1,2,3-de][1,4]benzoxacin-6-carbonsäure-Derivate gemäß Ansprüchen 1 bis 3 zur Bekämpfung von Krankheiten.

**6.** 7-Oxo-7H-pyrido[1,2,3-de][1,4]benzoxacin-6-carbonsäure-Derivate gemäß Ansprüchen 1 bis 3 zur Bekämpfung von Virusinfektionen.

**7.** 7-Oxo-7H-pyrido[1,2,3-de][1,4]benzoxacin-6-carbonsäure-Derivate gemaß Ansprüchen 1 bis 3 zur Bekämpfung von Hepatitis.

**8.** Arzneimittel enthaltend 7-Oxo-7H-pyrido[1,2,3-de][1,4]benzoxacin-6-carbonsäure-Derivate gemäß Ansprüchen 1 bis 3.

**9.** Antivirale Mittel enthaltend 7-Oxo-7H-pyrido[1,2,3-de][1,4]benzoxacin-6-carbonsäure-Derivate gemäß Ansprüchen 1 bis 3.

**10.** Verwendung von 7-Oxo-7H-pyrido[1,2,3-de][1,4]benzoxacin-6-carbonsäure-Derivaten gemäß Ansprüchen 1 bis 3 bei der Herstellung von Arzneimitteln.

Europäisches Patentamt

# EUROPÄISCHER RECHERCHENBERICHT

Nummer der Anmeldung

EP 93 10 4660

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 253 235 (BAYER AG)<br>* Ansprüche 1,8 *<br>--- | 1,8 | C07D498/06<br>A61K31/535<br>//(C07D498/06, 265:00,221:00) |
| D,X | JOURNAL OF HETEROCYCLIC CHEMISTRY.<br>Bd. 28, Nr. 4, 1991, PROVO US<br>Seiten 1067 - 1074<br>TETSUO OKADA ET AL 'Synthesis and antibacterial activities of novel oxazine and thiazine ring-fused tricyclic quinolonecarboxylic acids: 10-(alicyclic amino)-9-fluoro-7-oxo-7H-pyrido(1,2,3-de)(1,4)benzoxazine-6-carboxylic acids and the corresponding 1-thia congeners'<br>* das ganze Dokument *<br>----- | 1,8 | |
| | | | RECHERCHIERTE SACHGEBIETE (Int. Cl.5) |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 07 JULI 1993 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)